# EUROPEAN PATENT APPLICATION

(11) **EP 4 834 942 A1**
(43) Date of publication of application: **04.11.2026**
(21) Application number: 24918195.9
(22) Date of filing: 12.11.2024
(51) Int. Cl.: C07K 16/28, C07K 16/32, C07K 16/46, A61K 39/395, A61P 35/00

(54) **MONOCLONAL ANTIBODY SPECIFICALLY BINDING TO HEGFR AND BISPECIFIC ANTIBODY**

(30) Priority: 18.01.2024 CN 202410077500
(71) Applicant: Celest Therapeutics, Grand Cayman KY1-1003 (KY)
(72) Inventor: ZHANG, Fei, Shanghai 201315 (CN); WANG, Ying, Shanghai 201315 (CN); ZHANG, Man, Shanghai 201315 (CN); SHI, Dandan, Shanghai 201315 (CN); WU, Bo, Shanghai 201315 (CN)
(74) Representative: Canzler & Bergmeier Patentanwälte Partnerschaft mbB
(86) International application number: PCT/CN2024/131632
(87) International publication number: WO 2025/152586

(57) **Abstract**

The present application provides an antibody or antigen-binding fragment thereof specifically binding to hEGFR, and a bispecific antibody, antibody-drug conjugate and pharmaceutical composition comprising said antibody. Further, the present application also provides uses of the antibody or antigen-binding fragment thereof or the bispecific antibody.

## Description

### TECHNICAL FIELD

The present invention relates to the field of biomedicine, in particular relates to an antibody or antigen-binding fragment thereof specifically binding to hEGFR, and a bispecific antibody comprising the same.

### BACKGROUND

EGFR (Epidermal Growth Factor Receptor) is a member of the human epidermal growth factor receptor (HER) family, which is a Receptor Tyrosine Kinase (RTK) including HER1 (EGFR, ErbB1), HER2 (ErbB2), HER3 (ErbB3) and HER4 (ErbB4). EGFR is a single transmembrane glycoprotein, mainly composed of three parts: a ligand-binding extracellular domain, a hydrophobic transmembrane domain for receptor dimerization, and an intracellular kinase active domain. The activation process of the EGFR signaling pathway is as follows: ligand binding induces conformational changes of EGFR, EGFR homodimerizes or heterodimerizes with family members, and the intracellular kinase domain is phosphorylated, thereby initiating signal cascade transduction that ultimately affects cell function. EGFR is widely distributed on the surface of mammalian epithelial cells, fibroblasts, glial cells, keratinocytes and other cells, responsible for regulating cell growth, proliferation and differentiation. Studies have shown that EGFR is abnormally activated in many epithelial tumors, such as non-small cell lung cancer, breast cancer, colorectal cancer, head and neck cancer and glioblastoma. Overexpression of EGFR, gene amplification, mutation activation, overexpression of ligands and/or loss of EGFR activity regulators can all cause functional abnormalities of EGFR.

EGFR tyrosine kinase inhibitors (EGFR-TKI) mainly include small molecule compounds and antibodies. The commercialized antibodies include Cetuximab, Nimotuzumab, Panitumumab and Necitumumab. EGFR antibodies mainly achieve the effect of blocking signal transduction by competing with endogenous ligands for binding to EGFR and inhibiting dimerization between family members. Among them, Panitumumab is the only fully humanized monoclonal antibody with strong affinity for EGFR, reaching 10⁻¹¹ M. In 2006, FDA approved Panitumumab for the treatment of metastatic colorectal cancer after failure of chemotherapy. However, clinical practice has proven that only a small portion of patients can benefit from antibody therapy. For example, in colorectal cancer, although EGFR is highly expressed in 65% of patients, only about 10% of patients can delay disease progression. In addition, antibody drugs such as Panitumumab have a strong skin toxicity, which may be caused by a high affinity, and this problem limits the treatment population by the antibody. Finally, patients who benefit also develop drug resistance, which further reduces the effectiveness of the antibody. EGFR-TKI resistance includes EGFR-dependent resistance and EGFR-independent resistance. Among them, EGFR-independent resistance is mainly a bypass signal pathway activation, such as gene amplification or upregulated expression of HGF/c-MET, HER2, FGFR1, IGF1R, etc. Among them, c-MET gene amplification is the most common, accounting for 5%-10% of acquired resistance cases to EGFR-TKI. c-MET is also a transmembrane receptor tyrosine kinase that can bypass EGFR to activate the PI3K/AKT signaling pathway. In addition, abnormal activation of EGFR downstream signaling pathways, such as KRAS mutation activating the MAPK signaling pathway, PI3KCA mutation activating the PI3K/AKT/mTOR pathway, and PTEN loss activating the PI3K/AKT signaling pathway, are also important mechanisms of EGFR-independent resistance. In view of this, many companies have developed antibody drugs targeting bypass activation proteins such as c-Met and HGF. However, many drugs such as Onartuzumab (MetMab) and Tivantinib have failed in clinical trials due to insufficient efficacy.

Therefore, there is an urgent need in the art to introduce new drug mechanisms and forms, such as bispecific antibodies, to increase the treatment population, expand the therapeutic window, and improve the safety and efficacy of drugs. The advantage of bispecific antibody drugs is that the affinity and binding epitopes of each antibody arm can be fine-tuned according to antigen expression levels, and multiple antigens can be bridged to achieve optimal biological activity. For example, bispecific antibodies that simultaneously block EGFR and other bypass activation target signaling pathways are a very promising treatment method.

### CONTENT OF THE DISCLOSURE

The present invention provides a modified hEGFR antibody and/or antigen-binding fragment thereof, and a bispecific antibody comprising the hEGFR antibody and/or antigen-binding fragment thereof. Further, the present invention also provides uses of the antibody or antigen-binding fragment thereof or the bispecific antibody.

Specifically, the present invention relates to the following aspects:
In a first aspect, an antibody or antigen-binding fragment thereof specifically binding to human epidermal growth factor receptor (hEGFR), wherein the antibody or antigen-binding fragment comprises:
(a1) a heavy chain variable region (VH), comprising HCDR1, HCDR2 and HCDR3, which according to the Kabat sequence numbering system respectively comprise the amino acid sequences shown in SEQ ID NO: 7, SEQ ID NO: 2 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a2) a heavy chain variable region (VH), comprising HCDR1, HCDR2 and HCDR3, which according to the Kabat sequence numbering system respectively comprise the amino acid sequences shown in SEQ ID NO: 8, SEQ ID NO: 2 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a3) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 9 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a4) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 10 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a5) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 11 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a6) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 12 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a7) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 13 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b2) a light chain variable region (VL), comprising LCDR1, LCDR2 and LCDR3, which according to the Kabat sequence numbering system respectively comprise the amino acid sequences shown in SEQ ID NO: 14, SEQ ID NO: 5 and SEQ ID NO: 6 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b3) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 15 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b4) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 16 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b5) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 17 or variants thereof;
wherein the variants of CDRs have 3, 2 or 1 amino acid differences from, or have at least 80%, 85%, 90%, 91%, 92%, 93%, 94%, 95%, 96%, 97%, 98%, 99% identity to the corresponding CDRs, respectively.

In one embodiment, the amino acid differences described above include amino acid substitutions, deletions or additions; preferably, said substitutions are conservative substitutions.

In one embodiment, the antibody or antigen-binding fragment thereof described above, wherein the VH and VL are selected from the group consisting of, or variants thereof:
(a) a heavy chain having a variable domain comprising an amino acid sequence selected from any one of SEQ ID NOs: 20-26 and a light chain having a variable domain comprising the amino acid sequence shown in SEQ ID NO: 19; or
(b) a heavy chain having a variable domain comprising the amino acid sequence shown in SEQ ID NO: 18 and a light chain having a variable domain comprising an amino acid sequence selected from any one of SEQ ID NOs: 27-30;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

In one embodiment, the amino acid differences described above include amino acid substitutions, deletions or additions; preferably, said substitutions are conservative substitutions.

In one embodiment, the antibody or antigen-binding fragment thereof described above, wherein the antibody comprises a monoclonal antibody, a polyclonal antibody, a multispecific antibody, a chimeric antibody, a humanized antibody or a fully human antibody; and/or, the antigen-binding fragment comprises an Fab, an Fab', an Fv fragment, an F(ab')₂, an Fd fragment, a dAb, a complementarity determining region fragment, an scFv or a single-domain antibody.

In one embodiment, the antigen-binding fragment described above is an scFv, sequentially comprising a light chain variable region, a linking fragment and a heavy chain variable region from N-terminus to C-terminus.

In one embodiment, the linking fragment described above is selected from (GS)n, (GGS)n, (GGGS)n, or (GGGGS)n, wherein n is selected from 2, 3, or 4; preferably, said linking fragment comprises the amino acid sequence shown in SEQ ID NO: 78.

In one embodiment, the antigen-binding fragment described above is an scFv, comprising any one of the amino acid sequences shown in SEQ ID NOs: 32-42 or variants thereof, wherein the variants have 3, 2 or 1 amino acid differences from, or have at least 80%, 85%, 90%, 91%, 92%, 93%, 94%, 95%, 96%, 97%, 98%, 99% identity to said scFv, respectively.

In one embodiment, the amino acid differences described above include amino acid substitutions, deletions or additions; preferably, said substitutions are conservative substitutions.

In one embodiment, the scFv described above further comprises point mutations H44C and L100C to form a disulfide bond.

In one embodiment, the antibody or antigen-binding fragment thereof described above, wherein a maximum effect (Eₘₐₓ) induced by the binding of the antibody or antigen-binding fragment to an EGFR-expressing cell line is more than 10% lower than a maximum effect (Eₘₐₓ) induced by the binding of the parent antibody to said EGFR-expressing cell line. A non-limiting example of said EGFR-expressing cell line is A431 cell line.

In one embodiment, the antibody or antigen-binding fragment thereof described above inhibits Panitumumab binding to an EGFR-expressing cell line, such as the A431 cell line, with an IC₅₀ value higher than 2.10 nM.

In another aspect, the present invention also provides a multispecific antibody comprising a plurality of different antigen-binding domains, wherein a first antigen-binding domain comprises the antibody or antigen-binding fragment thereof described above.

In another aspect, the present invention also provides a bispecific antibody comprising two different antigen-binding domains, wherein a first antigen-binding domain comprises the antibody or antigen-binding fragment thereof described above, and a second antigen-binding domain comprises an antibody or antigen-binding fragment thereof specifically binding to a tumor-associated antigen (TAA).

In one embodiment, the tumor-associated antigen described above is selected from any one of the group consisting of c-MET, AFP, ALK, BAGE protein, BIRC5, BIRC7, β-catenin, brc-abl, BRCA1, BORIS, CA9, carbonic anhydrase IX, caspase-8, CALR, CCR5, CD19, CD20, CD22, CD30, CD40, CDK4, CEA, CTLA4, cyclin-B1, CYP1B1, EGFRvIII, ErbB3, ErbB4, ETV6-AML, EpCAM, EphA2, Fra-1, FOLR1, GAGE protein, GD2, GD3, GloboH, glypican-3, GM3, gp100, Her2, HLA/B-raf, HLA/k-ras, HLA/MAGE-A3, hTERT, LMP2, MAGE protein, MART-1, mesothelin, ML-IAP, Muc1, Muc2, Muc3, Muc4, Muc5, Muc16, MUM1, NA17, NY-BR1, NY-BR62, NY-BR85, NY-ESO1, OX40, p15, p53, PAP, PAX3, PAX5, PCTA-1, PLAC1, PRLR, PRAME, PSMA, RAGE protein, Ras, RGS5, Rho, SART-1, SART-3, STEAP1, STEAP2, TAG-72, TGF-β, TMPRSS2, Thomson-Friedenreich antigen, TRP-1, TRP-2, tyrosinase and uroplakin-3.

In one embodiment, the second antigen-binding domain described above specifically binds to c-MET.

In one embodiment, the second antigen-binding domain specifically binding to c-MET described above comprises:
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b1) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b2) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 49 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b3) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 50 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b4) a light chain variable region (VL), comprising LCDR1 region, LCDR2 region and LCDR3 region, which according to the Kabat sequence numbering system respectively comprise the amino acid sequences shown in SEQ ID NO: 51, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b5) a light chain variable region (VL), comprising LCDR1 region, LCDR2 region and LCDR3 region, which according to the Kabat sequence numbering system respectively comprise the amino acid sequences shown in SEQ ID NO: 52, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b6) a light chain variable region (VL), comprising LCDR1 region, LCDR2 region and LCDR3 region, which according to the Kabat sequence numbering system respectively comprise the amino acid sequences shown in SEQ ID NO: 53, SEQ ID NO: 47 and SEQ ID NO: 48 or variants thereof;
wherein the variants of CDRs have 3, 2 or 1 amino acid differences from, or have at least 80%, 85%, 90%, 91%, 92%, 93%, 94%, 95%, 96%, 97%, 98%, 99% identity to the corresponding CDRs, respectively.

In one embodiment, the amino acid differences described above include amino acid substitutions, deletions or additions; preferably, said substitutions are conservative substitutions.

In one embodiment, the second antigen-binding domain specifically binding to c-MET described above has:
1) a heavy chain comprising a variable domain having the amino acid sequence shown in SEQ ID NO: 54 or variants thereof; and
2) a light chain comprising a variable domain having an amino acid sequence selected from any of SEQ ID NOs: 55-60 or variants thereof;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

In one embodiment, the amino acid differences described above include amino acid substitutions, deletions or additions; preferably, said substitutions are conservative substitutions.

In one embodiment, the second antigen-binding domain described above further comprises a first heavy chain constant region (CH1) and a light chain constant region (CL).

In one embodiment, the CH1 described above comprises the amino acid sequence shown in SEQ ID NO: 75, and the CL comprises the amino acid sequence shown in SEQ ID NO: 76 or 77.

In one embodiment, the second antigen-binding domain described above consists of a light chain-heavy chain pair, wherein the light chain comprises any one of the amino acid sequences shown in SEQ ID NOs: 63-67, and the heavy chain comprises the amino acid sequence shown in SEQ ID NO: 61.

In one embodiment, the second antigen-binding domain described above comprises an Fab, an Fab', an Fv fragment, an F(ab')₂, an Fd fragment, a dAb, a complementarity determining region fragment, an scFv or a single-domain antibody.

In one embodiment, the bispecific antibody described above further comprises an Fc domain.

In one embodiment, the Fc domain described above is selected from an IgA Fc fragment, an IgG Fc fragment, an IgM Fc fragment, an IgD Fc fragment, or an IgE Fc fragment; said IgG Fc fragment is preferably an IgG1 Fc fragment, an IgG2 Fc fragment, an IgG3 Fc fragment, or an IgG4 Fc fragment; said Fc domain is preferably an IgG1 Fc fragment comprising the amino acid sequence shown in SEQ ID NO: 79.

In one embodiment, the Fc domain described above further comprises amino acid substitutions that promote pairing of a first subunit and a second subunit of said Fc domain.

In one embodiment, the first subunit of said Fc domain described above comprises amino acid substitutions: Y349C/T366S/L368A/Y407V, and the second subunit of said Fc domain comprises amino acid substitutions:
S354C/T366W.

In one embodiment, the N-terminus of said Fc domain described above is fused to the C-terminus of the first antigen-binding domain, and/or the N-terminus of said Fc domain is fused to the C-terminus of the heavy chain of the second antigen-binding domain.

In one embodiment, the bispecific antibody described above is selected from the group consisting of, or variants thereof:
(a) a first antigen-binding domain comprising HCDR1-3 with the sequences shown in SEQ ID NOs: 1-3 and LCDR1-3 with the sequences shown in SEQ ID NOs: 4-6; or
(b) a first antigen-binding domain comprising HCDR1-3 with the sequences shown in SEQ ID NOs: 8, 2 and 3 and LCDR1-3 with the sequences shown in SEQ ID NOs: 4-6; or
(c) a first antigen-binding domain comprising HCDR1-3 with the sequences shown in SEQ ID NOs: 1-3 and LCDR1-3 with the sequences shown in SEQ ID NOs: 4, 5, and 15; or
(d) a first antigen-binding domain comprising HCDR1-3 with the sequences shown in SEQ ID NOs: 7, 2, and 3 and LCDR1-3 with the sequences shown in SEQ ID NOs: 4, 5, and 15; or
(e) a first antigen-binding domain comprising HCDR1-3 with the sequences shown in SEQ ID NOs: 1, 10 and 3 and LCDR1-3 with the sequences shown in SEQ ID NOs: 4-6; or
(f) a first antigen-binding domain comprising HCDR1-3 with the sequences shown in SEQ ID NOs: 1, 2 and 12 and LCDR1-3 with the sequences shown in SEQ ID NOs: 4-6;

and a second antigen-binding domain comprising HCDR1-3 with the sequences shown in SEQ ID NOs: 43-45 and LCDR1-3 with the sequences shown in SEQ ID NOs: 46-48;
wherein the variants have 3, 2 or 1 amino acid differences from, or have at least 80%, 85%, 90%, 91%, 92%, 93%, 94%, 95%, 96%, 97%, 98%, 99% identity to the corresponding CDRs, respectively.

In one embodiment, the amino acid differences described above include amino acid substitutions, deletions or additions; preferably, said substitutions are conservative substitutions.

In one embodiment, the bispecific antibody described above is selected from the group consisting of, or variants thereof:
(a) a first antigen-binding domain comprising VH of the sequence shown in SEQ ID NO: 18 and VL of the sequence shown in SEQ ID NO: 19; or
(b) a first antigen-binding domain comprising VH of the sequence shown in SEQ ID NO: 21 and VL of the sequence shown in SEQ ID NO: 19; or
(c) a first antigen-binding domain comprising VH of the sequence shown in SEQ ID NO: 18 and VL of the sequence shown in SEQ ID NO: 28; or
(d) a first antigen-binding domain comprising VH of the sequence shown in SEQ ID NO: 20 and VL of the sequence shown in SEQ ID NO: 28; or
(e) a first antigen-binding domain comprising VH of the sequence shown in SEQ ID NO: 23 and VL of the sequence shown in SEQ ID NO: 19; or
(f) a first antigen-binding domain comprising VH of the sequence shown in SEQ ID NO: 25 and VL of the sequence shown in SEQ ID NO: 19;

and a second antigen-binding domain comprising VH of the sequence shown in SEQ ID NO: 54 and VL of the sequence shown in SEQ ID NO: 55;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

In one embodiment, the amino acid differences described above include amino acid substitutions, deletions or additions; preferably, said substitutions are conservative substitutions.

In one embodiment, the bispecific antibody described above consisting of a first heavy chain comprising the first antigen-binding domain and a second heavy chain comprising the second antigen-binding domain, and a first light chain; wherein the first heavy chain is selected from any one of the amino acid sequences shown in SEQ ID NOs: 69-74 or variants thereof, the second heavy chain has the sequence shown in SEQ ID NO: 68 or variants thereof, and the first light chain has the sequence shown in SEQ ID NO: 62 or variants thereof;
wherein the variants have 3, 2 or 1 amino acid differences from, or have at least 80%, 85%, 90%, 91%, 92%, 93%, 94%, 95%, 96%, 97%, 98%, 99% identity to the corresponding sequences, respectively.

In one embodiment, the amino acid differences described above include amino acid substitutions, deletions or additions; preferably, said substitutions are conservative substitutions.

In one embodiment, the bispecific antibody described above can simultaneously bind to EGFR and c-MET, and bind to EGFR and c-MET co-expressing cell lines, such as H1975 or H441 cell lines, with an EC₅₀ value lower than 10 nM.

In one embodiment, the bispecific antibody described above binds to EGFR high-expressing cell lines, such as A431 cell lines or keratinocytes, with an EC₅₀ value higher than 50 nM.

In one embodiment, the bispecific antibody described above is preferentially internalized by EGFR and c-MET co-expressing cell lines when EGFR and c-MET co-expressing cell lines and EGFR high-expressing cell lines are present simultaneously. For example, when H1975 cells and keratinocytes are present simultaneously, said bispecific antibody is preferentially internalized by H1975 cells. This indicates that the bispecific antibody has an obvious selectivity i*n in vitro* cytotoxicity killing experiments.

In another aspect, the present invention also provides a nucleic acid molecule encoding the antibody or antigen-binding fragment thereof specifically binding to human epidermal growth factor receptor (hEGFR) described above or the bispecific antibody described above.

In another aspect, the present invention also provides an expression vector comprising the nucleic acid molecule described above.

In another aspect, the present invention also provides a host cell comprising the nucleic acid molecule described above or the expression vector described above, or expressing the antibody or antigen-binding fragment thereof described above or the multispecific antibody described above or the bispecific antibody described above.

In another aspect, the present invention also provides an antibody-drug conjugate (ADC), consisting of an antibody, a linker and a payload, wherein the antibody is the antibody or antigen-binding fragment thereof specifically binding to human epidermal growth factor receptor (hEGFR) described above or the multispecific antibody described above or the bispecific antibody described above.

In another aspect, the present invention also provides a pharmaceutical composition, comprising the antibody or antigen-binding fragment thereof specifically binding to human epidermal growth factor receptor (hEGFR) described above or the multispecific antibody described above or the bispecific antibody described above or the antibody-drug conjugate described above, and at least one pharmaceutically acceptable carrier and/or excipient.

In another aspect, the present invention also provides a method for inhibiting tumor cell growth and/or killing said tumor cells, comprising contacting said tumor cells with an effective amount of the antibody or antigen-binding fragment thereof described above or the multispecific antibody described above or the bispecific antibody described above or the antibody-drug conjugate described above or the pharmaceutical composition described above.

In another aspect, the present invention also provides the antibody or antigen-binding fragment thereof described above or the multispecific antibody described above or the bispecific antibody described above or the antibody-drug conjugate described above or the pharmaceutical composition described above for use in the manufacture of a medicament for the treatment of a tumor.

In one embodiment, according to the application described above, wherein the tumor is selected from pancreatic cancer, melanoma, glioblastoma, head and neck cancer, prostate cancer, osteosarcoma, colorectal cancer, gastric cancer, malignant mesothelioma, multiple myeloma, ovarian cancer, non-small cell lung cancer, synovial sarcoma, thyroid cancer, breast cancer, squamous cell carcinoma, esophageal cancer, clear cell renal cell carcinoma, chromophobe renal cell carcinoma, renal oncocytoma, renal transitional cell carcinoma, urothelial carcinoma, adenocarcinoma or small cell carcinoma.

In another aspect, the present invention also provides a method for treating a tumor in a subject, said method comprising administering an effective amount of the antibody or antigen-binding fragment thereof described above, or the multispecific antibody or bispecific antibody described above, or the antibody-drug conjugate described above, or the pharmaceutical composition described above to a subject in need thereof.

In one embodiment, the tumor is selected from pancreatic cancer, melanoma, glioblastoma, head and neck cancer, prostate cancer, osteosarcoma, colorectal cancer, gastric cancer, malignant mesothelioma, multiple myeloma, ovarian cancer, non-small cell lung cancer, synovial sarcoma, thyroid cancer, breast cancer, squamous cell carcinoma, esophageal cancer, clear cell renal cell carcinoma, chromophobe renal cell carcinoma, renal oncocytoma, renal transitional cell carcinoma, urothelial carcinoma, adenocarcinoma or small cell carcinoma.

In one embodiment, the subject is a mammal, preferably a human.

In one embodiment, the method further comprises co-administering other drugs having an antitumor activity, said other drugs having an antitumor activity including but not limited to alkylating agents, mitotic inhibitors, antitumor antibiotics, antimetabolites, topoisomerase inhibitors, tyrosine kinase inhibitors, radionuclide agents, radiosensitizers, anti-angiogenic agents, cytokines, molecular targeted drugs, immune checkpoint inhibitors or oncolytic viruses.

In one embodiment, the method further comprises administering additional antitumor therapy, said additional antitumor therapy including but not limited to surgery, radiotherapy, targeted therapy, immunotherapy, hormone therapy, gene therapy or palliative care.

The present invention provides an antibody or antigen-binding fragment thereof specifically binding to hEGFR, and a bispecific antibody, an antibody-drug conjugate and a pharmaceutical composition comprising said antibody. Further, the present application also provides uses of the antibody or antigen-binding fragment thereof or the bispecific antibody. Compared with the wild-type antibody, the bispecific antibody provided by the present invention can promote the internalization and degradation of EGFR and c-MET antigens and inhibit EGFR downstream signal transduction. It should be particularly noted that the bispecific antibody provided by the present invention has a selective activity against cell lines with different expression levels of EGFR and c-MET, and has a good activity sparing for human keratinocytes with a high expression of EGFR but a low expression of c-MET, which can overcome the skin side reactions and ocular toxicity commonly caused by clinically available EGFR-targeted drugs, and has potential clinical safety advantages. Therefore, the bispecific antibody provided by the present application has a significant clinical value.

It should be understood that within the scope of the present invention, the above-mentioned technical features of the present invention and the technical features specifically described below (such as in the examples) can be combined with each other to constitute new or preferred technical solutions. Limited by space, these will not be enumerated one by one herein.

In the present invention, unless otherwise specified, the terms involved herein have conventional meanings understood by those skilled in the art. Where a term has two or more definitions as used and/or accepted in the art, the definition of the term as used herein is intended to include all such meanings. The molecular genetics, nucleic acid chemistry, chemistry, molecular biology, biochemistry, cell culture, microbiology, cell biology, genomics and recombinant DNA operations described herein are all conventional steps widely used in the corresponding fields. To better understand the present invention, definitions and explanations of relevant terms are provided below.

Those of ordinary skill in the art can understand that the CDR region of an antibody is responsible for the binding specificity of the antibody to the antigen. In the case where the sequences of heavy chain and light chain variable regions of an antibody are known, there are currently several methods for determining the CDR region of an antibody, including the Kabat, IMGT, Chothia and AbM numbering systems. However, the application of each definition of the CDR of an antibody or variant thereof will be within the scope of the terms defined and used herein. Given the amino acid sequence of the variable region of an antibody, those skilled in the art can generally determine the specific CDR without relying on any experimental data beyond the sequence itself.

As used herein, "antibody" or "antigen-binding fragment" refers to a polypeptide or polypeptide complex that specifically recognizes and binds to an antigen. The term "antibody" is used in a broad sense and includes immunoglobulins or antibody molecules, said antibody molecules including monoclonal or polyclonal human, humanized, composite and chimeric antibodies and antibody fragments. Thus the term "antibody" includes any protein or peptide containing a specific molecule that contains at least a portion of an immunoglobulin molecule having biological activity of binding to an antigen. Examples of such cases include, but are not limited to, complementarity determining regions (CDRs) of heavy or light chains or their ligand-binding portions, heavy or light chain variable regions, heavy or light chain constant regions, framework (FR) regions or any portion thereof, or at least a portion of a binding protein. In the present invention, antibodies include murine, chimeric, humanized or fully human antibodies prepared by techniques well known to those skilled in the art. Recombinant antibodies, such as chimeric and humanized monoclonal antibodies, including human and non-human portions, can be prepared using DNA recombination techniques well known in the art. The immunoglobulin molecule or antibody molecule of the present application may be any type (e.g., IgG, IgE, IgM, IgD, IgA and IgY), any class of immunoglobulin molecule (e.g., IgG1, IgG2, IgG3, IgG4, IgA1 and IgA2) or subclass.

As used herein, "antibody fragment" or "antigen-binding fragment" includes but is not limited to F(ab')₂, Fab', Fab, Fv, Fd, dAb, Fab/c, complementarity determining region (CDR) fragments, single chain Fvs (ScFv), disulfide-stabilized Fv (Disulfide-stabilized Fv fragment, dsFv), (dsFv)₂, bispecific dsFv (dsFv-dsFv), diabody, disulfide-stabilized diabody (ds-Diabody), ScFv multimers (such as ScFv dimers, ScFv trimers), multispecific antibodies formed by a portion of an antibody comprising one or more CDRs, nanobodies, single-domain antibodies (sdAb), domain antibodies, bivalent domain antibodies, or any other antibody fragments that bind to an antigen but do not comprise a complete antibody structure. Regardless of structure, antigen-binding fragments include any polypeptide or polypeptide complex capable of binding to the same antigen as the parent antibody or parent antibody fragment. As used herein, "antibody fragment" includes aptamers, spiegelmers and diabodies. As used herein, "antibody fragment" also includes any synthetic or genetically engineered protein that, like an antibody, can bind to a specific antigen to form a complex. Typically, antibody fragments have at least about 50 contiguous amino acids of the antibody of the present invention, preferably at least about 50 contiguous amino acids, more preferably at least about 80 contiguous amino acids, and most preferably at least about 100 contiguous amino acids.

As used herein, "Fd" means an antibody fragment composed of VH and CH1 domains; the term "dAb fragment" means an antibody fragment composed of a VH domain (Ward et al., Nature 341:544-546 (1989)); the term "Fab fragment" means an antibody fragment composed of VL, VH, CL and CH1 domains; the term "F(ab')₂ fragment" means an antibody fragment comprising two Fab fragments connected by a disulfide bridge on the hinge region; the term "Fab' fragment" means a fragment obtained after reducing the disulfide bond that connects the two heavy chain fragments in the F(ab')₂ fragment, composed of one complete light chain and an Fd fragment of the heavy chain (composed of VH and CH1 domains).

As used herein, "Fv" means an antibody fragment composed of the VL and VH domains of a single arm of an antibody. The Fv fragment is generally considered to be the smallest antibody fragment capable of forming a complete antigen-binding site. It is generally believed that six CDRs confer antigen-binding specificity to an antibody. However, even a single variable region (such as an Fd fragment, which contains only three CDRs specific for an antigen) can recognize and bind to an antigen, although its affinity may be lower than that of a complete binding site.

As used herein, "Fc" means an antibody fragment formed by the second and third constant regions of the first heavy chain and the second and third constant regions of the second heavy chain of an antibody bound together by a disulfide bond. The Fc fragment of an antibody has various different functions but does not participate in antigen binding.

As used herein, "single chain variable fragment" or "ScFv" refers to a fusion protein of the variable regions of the heavy chain (VH) and light chain (VL) of an immunoglobulin. In certain aspects, these regions are connected by a short linker peptide of 10 to about 25 amino acids. The linker may be rich in glycine for flexibility, and also contain serine or threonine for solubility, and can connect the N-terminus of VH to the C-terminus of VL, and vice versa. The protein retains the characteristics of the original immunoglobulin, except that the constant region is removed and a linker is introduced. ScFv molecules are known in the art. In some cases, a disulfide bond may also exist between the VH and VL of an scFv.

As used herein, "single-domain antibody (sdAb)" has the meaning generally understood by those skilled in the art, and refers to an antibody fragment composed of a single monomeric variable antibody domain (such as a single heavy chain variable region) that maintains the ability to specifically bind to the same antigen as a full-length antibody. Single-domain antibodies are also called nanobodies.

Each of the above antibody fragments maintains the ability to specifically bind to the same antigen as a full-length antibody, and/or competes with the full-length antibody for specific binding to the antigen.

Antigen-binding fragments of an antibody (e.g., the above antibody fragments) can be obtained from a given antibody (e.g., the antibody provided by the present invention) using conventional techniques known to those skilled in the art (e.g., recombinant DNA techniques or enzymatic or chemical cleavage methods), and screened for specificity in the same manner as for intact antibodies.

In the present disclosure, unless the context clearly indicates otherwise, when the term "antibody" is mentioned, it includes not only intact antibodies but also antigen-binding fragments of antibodies.

As used herein, "chimeric antibody" refers to an antibody in which a portion of the light chain and/or heavy chain is derived from one antibody (which may be derived from a particular species or belong to a particular antibody class or subclass), and another portion of the light chain and/or heavy chain is derived from another antibody (which may be derived from the same or different species or belong to the same or different antibody class or subclass), but in any case retains binding activity to the target antigen. In certain embodiments, the term "chimeric antibody" may include an antibody in which the heavy and light chain variable regions of the antibody are from a first antibody, and the heavy and light chain constant regions of the antibody are from a second antibody.

As used herein, "bispecific antibody" or "bispecific" refers to a conjugate formed by a first antibody (or fragment thereof) and a second antibody (or fragment thereof) or antibody analog through a coupling arm, the coupling methods including but not limited to chemical reaction, gene fusion and enzymatic methods. Bispecific antibodies can be connected or produced by various methods, such as the methods of Songsivilai et al. (Clin. Exp. Immunol., 79:315-321 (1990)), and the methods of Kostelny et al. (J. Immunol., 148:1547-1553 (1992)).

The antigen-binding domain of the antibody of the present invention is Fab, or ScFv, or non-covalent pairing between heavy chain variable region (VH)-light chain variable region (VL) (Fv). Any of the above antibodies or polypeptides may also include additional polypeptides, such as a signal peptide at the N-terminus of the antibody for directing secretion, or a 6×His tag for purification. The present invention includes not only intact antibodies but also antibody fragments having an immunological activity or fusion proteins formed by antibodies and other sequences.

The present invention also includes fragments, variants, derivatives and analogs of said antibodies. The antibodies, antigen-binding fragments, variants or derivatives of the present application include, but are not limited to, polyclonal antibodies, monoclonal antibodies, multispecific antibodies (such as bispecific antibodies, trispecific antibodies, etc.), human antibodies, animalderived antibodies, humanized antibodies, primatized antibodies, or chimeric antibodies, CDR-grafted and/or modified antibodies, single chain antibodies (e.g., ScFv), diabodies, epitope-binding fragments, such as Fab, Fab' and F(ab')₂, Fd, Fv, single chain Fv (ScFv), single chain antibodies, disulfidelinked Fv (dsFv), fragments comprising VL domains or VH domains, fragments produced by Fab expression libraries, and anti-idiotypic (anti-Id) antibodies. The antibody fragments, antigen-binding fragments, derivatives or analogs of the present invention may also be polypeptides in which one or more conservative or non-conservative amino acid residues (preferably conservative amino acid residues) have been substituted.

Amino acid numbering follows the Kabat numbering. "Kabat numbering" refers to the numbering system described by Kabat et al., the content of which is recorded in "Sequence of Proteins of Immunological Interest" by the U.S. Department of Health and Human Services (1983).

The antibody can be modified to improve heterodimer pairing efficiency. For example, in certain aspects, compared with wild-type antibody fragments, the Fc fragment of the monovalent unit heavy chain and/or the Fc fragment of the fusion peptide may comprise one or more substitutions that form a knob-into-hole structural pair. The knob-into-hole configuration is known in the art.

As used herein, "identity" is used to refer to the sequence matching between two polypeptides or between two nucleic acids. To determine the percentage identity between two amino acid sequences or two nucleic acid sequences, the sequences are aligned for optimal comparison purposes (for example, gaps may be introduced in the first amino acid sequence or nucleic acid sequence for optimal alignment with the second amino acid or nucleic acid sequence). Then the amino acid residues or nucleotides at corresponding amino acid positions or nucleotide positions are compared. When a position in the first sequence is occupied by the same amino acid residue or nucleotide as the corresponding position in the second sequence, then the molecules are identical at that position. The percentage identity between two sequences is a function of the number of identical positions shared by the sequences (i.e., percentage identity = number of identical overlapping positions / total number of positions × 100%). In certain embodiments, the two sequences are of the same length.

The determination of percentage identity between two sequences can also be accomplished using a mathematical algorithm. A non-limiting example of a mathematical algorithm for comparison of two sequences is the algorithm of Karlin and Altschul, 1990, Proc. Natl. Acad. Sci. U.S.A. 87:2264-2268, as modified in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403.

As used herein, "variant", in the context of polypeptides (including polypeptides), also refers to polypeptides or peptides comprising amino acid sequences that have been altered by the introduction of substitutions, deletions or additions of amino acid residue. In certain cases, the term "variant" also refers to polypeptides or peptides that have been modified (i.e., by covalent attachment of any type of molecule to the polypeptide or peptide). For example, but not limited to, polypeptides may be modified, such as by glycosylation, acetylation, pegylation, phosphorylation, amidation, derivatization by known protecting/blocking groups, proteolytic cleavage, linkage to cell ligands or other proteins, etc. Derivative polypeptides or peptides can be produced by chemical modification using techniques known to those skilled in the art, including but not limited to specific chemical cleavage, acetylation, formylation, metabolic synthesis of tunicamycin, etc. Furthermore, variants have functions similar, identical or improved to those of the polypeptides or peptides from which they are derived.

As used herein, "conservative substitution" means an amino acid substitution that does not adversely affect or alter the expected properties of a protein/polypeptide comprising the amino acid sequence. For example, conservative substitutions can be introduced by standard techniques known in the art such as site-directed mutagenesis and PCR-mediated mutagenesis. Conservative amino acid substitutions include substitutions in which an amino acid residue is replaced by an amino acid residue having a similar side chain, such as substitution with a residue that is physically or functionally similar to the corresponding amino acid residue (e.g., having similar size, shape, charge, chemical properties, including the ability to form covalent bonds or hydrogen bonds, etc.). Families of amino acid residues having similar side chains have been defined in the art. These families include amino acids with basic side chains (e.g., lysine, arginine and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to substitute the corresponding amino acid residue with another amino acid residue from the same side chain family. Methods for identifying conservative amino acid substitutions are well known in the art.

As used herein, "Eₘₐₓ" or "maximum effect" refers to an increase in effect intensity with increasing drug dose or concentration within a certain range. However, when the effect increases to the maximum, continuing to increase the dose or concentration does not enhance the effect further. This limit of pharmacological effect is called the maximum effect. Eₘₐₓ reflects the intrinsic activity of a drug, which is the ability of a drug to produce an effect.

As used herein, "EC₅₀" or "half maximal effective concentration" refers to a drug dose corresponding to 50% of the maximum effect. It reflects the affinity of the drug for the target.

As used herein, "vector" refers to a nucleic acid vehicle into which a polynucleotide can be inserted. When a vector enables expression of a protein encoded by the inserted polynucleotide, the vector is called an expression vector. Vectors can be introduced into host cells by transformation, transduction or transfection, so that the genetic material elements they carry are expressed in the host cells. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); and phages such as lambda phage or M13 phage and animal viruses, etc. Animal viruses that can be used as vectors include but are not limited to, retroviruses (including lentiviruses), adenoviruses, adeno-associated viruses, herpesviruses (such as herpes simplex virus), poxviruses, baculoviruses, papillomaviruses, and papovaviruses (such as SV40). A vector may contain various elements that control expression, including but not limited to, promoter sequences, transcription initiation sequences, enhancer sequences, selectable elements and reporter genes. In addition, the vector may also contain a replication origin.

As used herein, "host cell" refers to a cell that can be used to introduce a vector, including but not limited to, prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, COS cells, NSO cells, HeLa cells, BHK cells, HEK 293 cells or human cells.

It should be noted that indefinite quantity modifiers of entities should refer to one or more (kinds of) said entities; for example, "multifunctional antibody" should be understood to mean one or more (kinds of) multifunctional antibodies. Likewise, indefinite quantity modifiers, the terms "one or more" and "at least one" are used interchangeably herein.

As used herein, "pharmaceutical composition" is a product that optionally contains a specific amount of one or more active ingredients (e.g., antibodies, small molecule drugs), and any product directly or indirectly produced by combining one or more active ingredients in optionally specific amounts. The different active ingredients in a pharmaceutical composition can be administered separately as separate preparations, including simultaneous administration or administration at different time points for synergistic effects. In the present disclosure, "pharmaceutical composition" and "formulation" are not mutually exclusive.

As used herein, "pharmaceutically acceptable carrier and/or excipient" refers to carriers and/or excipients that are pharmacologically and/or physiologically compatible with the subject and active ingredients, which are well known in the art.

As used herein, "treatment" refers to therapeutic treatment and preventive or prophylactic measures, wherein a subject is treated to prevent or slow (alleviate) adverse physiological changes or diseases, such as the development of cancer. Beneficial or desired clinical results include, but are not limited to, alleviation of symptoms, reduction of the extent of the disease, stabilization (e.g., preventing worsening) of the disease state, delay or slowing of disease progression, improvement or palliation of the disease state, and remission (whether partial or total), whether detectable or not. "Treatment" can also refer to prolonging survival compared to the expected survival without treatment. Conditions that require treatment include those that already have the condition or symptoms and those that are prone to have the condition or symptoms or those that will prevent the condition or symptoms.

As used herein, "subject" or "individual" or "animal" or "patient" or "mammal" refers to any subject, particularly mammalian subjects, that require diagnosis, prognosis or treatment. Mammalian subjects include humans, domestic animals, farm animals, zoo, sports, or pet animals, such as dogs, cats, guinea pigs, rabbits, rats, mice, horses, cattle, cows, primates (e.g., humans, monkeys such as cynomolgus monkeys, rhesus monkeys, baboons, and chimpanzees, etc.) and the like.

As used herein, "effective amount" refers to an amount sufficient to obtain or at least partially obtain the desired effect. For example, a disease-preventing effective amount refers to an amount sufficient to prevent, block, or delay the occurrence of said disease; a disease-treating effective amount refers to an amount sufficient to cure or at least partially prevent the disease and its complications in a patient already suffering from the disease. Determining such effective amounts is entirely within the capabilities of those skilled in the art. For example, the effective amount for therapeutic use will depend on the severity of the disease to be treated, the general state of the patient's own immune system, the general condition of the patient such as age, weight and gender, the mode of administration of the drug, and other treatments administered simultaneously, etc.

As described herein, the antigen-binding polypeptides, variants or derivatives of the present application can be used in certain therapeutic and diagnostic methods related to cancer or infectious diseases. The present application also relates to antibody-based therapies, which include administering the bispecific antibodies of the present application to a patient, such as an animal, mammal and human, for treating one or more diseases or conditions described herein. The therapeutic drugs of the present application include, but are not limited to, the antibodies of the present application (including variants and derivatives thereof as described herein) and nucleic acids or polynucleotides encoding the antibodies of the present application (including variants and derivatives thereof as described herein). The antibodies of the present application can also be used to treat, inhibit or prevent diseases, disorders or conditions, including malignant diseases, disorders, or conditions related to such diseases or disorders, such as immune response-related diseases. In some embodiments, the antibodies of the present invention can be used as immune-suppressants. In some embodiments, the antibodies of the present invention can be used to treat autoimmune diseases. The antigen-binding polypeptides, variants or derivatives of the present application are used to inhibit the growth, development and/or metastasis of cancer, particularly those listed above or in the paragraphs below.

Other diseases or conditions related to increased cell survival that can be treated, prevented, diagnosed and/or predicted with the antibodies or variants or derivatives thereof of the present application include, but are not limited to, cancer or tumors, including the development and/or metastasis of malignant tumors, and related diseases (such as malignant ascites, malignant pleural effusion, malignant effusion), such as EGFR-positive tumors.

Methods for administering the antibody, variants or derivatives thereof include, but are not limited to, intradermal, intramuscular, intraperitoneal, intravenous, subcutaneous, intranasal, epidural and oral routes. The antibody or composition can be administered by any convenient route, such as by infusion or bolus injection, by absorption through epithelial or mucosal linings of the skin (e.g., oral mucosa, rectal and intestinal mucosa, etc.), and can be administered together with other biologically active agents. Therefore, the pharmaceutical compositions containing antibodies of the present application can be administered orally, rectally, parenterally, intracisternally, intravaginally, intraperitoneally, topically (such as by powders, ointments, drops or transdermal patches), buccally, or as an oral or nasal spray. As used herein, the term "parenteral" refers to modes of administration including intravenous, intramuscular, intraperitoneal, sternal, subcutaneous and intra-articular injection and infusion. Administration can be systemic or local. It may also be desirable to administer the antigen-binding polypeptides or compositions of the present application locally to the area to be treated, which can be achieved by, for example but not limited to, local perfusion during surgery, topical application, such as in combination with postoperative wound dressings, by injection, by catheter, by suppository, or by implant, said implant being porous, non-porous, or gel-like material, including membranes or fibers. Preferably, when proteins (including antibodies) of the present application are administered, it must take care to use materials that do not absorb proteins.

### BRIEF DESCRIPTION OF THE DRAWINGS

Figure 1: Schematic diagram of exemplary anti-EGFR and c-MET bispecific antibodies. Figure 1A shows an "F(ab')₂ fragment" form expressed in a cell-free system. Figure 1B shows a structural form of bispecific antibodies expressed in a eukaryotic system.
Figure 2: Determination of binding activity of EGFR mutant antibodies to A431 cells.
Figure 3: Detection of competitive binding activity of EGFR mutant antibodies to parent antibodies by a flow cytometry.
Figure 4: Detection of binding activity of bispecific F(ab')₂ fragments to H1975 cell lines by a flow cytometry.
Figure 5: Detection of binding activity of bispecific F(ab')₂ fragments to H441 cell lines by a flow cytometry.
Figure 6: Detection of binding activity of bispecific F(ab')₂ fragments to A431 cell lines by a flow cytometry.
Figure 7: Detection of simultaneous binding capability of bispecific F(ab')₂ fragments to EGFR and c-MET by an ELISA bridging assay.
Figure 8: Detection of target internalization activity induced by bispecific F(ab')₂ fragments in H1975 cell lines by a fluorescence labeling.
Figure 9: Detection of target internalization activity induced by bispecific F(ab')₂ fragments in H441 cell lines by a fluorescence labeling.
Figure 10: Detection of target internalization activity induced by bispecific F(ab')₂ fragments in A431 cell lines by a fluorescence labeling.
Figure 11: Detection of affinity activity of the bispecific antibodies to H1975, H441 and A431 cell lines by a flow cytometry. Figures 11A-B are H1975 cell lines, Figure 11C is H441 cell lines, and Figure 11D is A431 cells.
Figure 12: Detection of target internalization activity induced by the bispecific antibodies in different tumor cell lines by a fluorescence labeling.
Figure 13: Detection of internalization activity induced by the bispecific antibodies in human primary keratinocyte cell lines by a fluorescence labeling.
Figure 14: Detection of target internalization activity induced by the bispecific antibodies in different tumor cell lines by an αFc-MMAE toxin secondary antibody method.
Figure 15: Detection of target internalization activity induced by the bispecific antibodies in different tumor cell lines by a DT3C toxin killing method.
Figure 16: Detection of activity of the bispecific antibodies in reducing EGFR and c-MET proteins in H1975 cells by an ELISA method.
Figure 17: Detection of inhibitory activity of the bispecific antibodies on downstream pERK and pAKT signals in EGF-activated H1975 cells by Western blot method.
Figure 18: Detection of selective killing activity of the bispecific antibodies on A431 cells and H1975 cells in mixed culture.

### DETAILED DESCRIPTION OF THE PREFERRED EMBODIMENT

The methods and applications of the present invention are described below in combination with the drawings, and the examples given are only for explaining the present invention and are not intended to limit the scope of the present invention. For those of ordinary skill in the art to which the present invention pertains, various simple deductions or substitutions can be made without departing from the spirit of the present invention, and all should be regarded as belonging to the protection scope of the present invention. Unless otherwise specified, the experiments and methods described in the examples are basically carried out according to conventional methods well known in the art and described in various references. Examples without specific conditions are carried out according to conventional conditions or conditions recommended by the manufacturer. Reagents or instruments without indicated manufacturers are all conventional products available through commercial purchase.

### Example 1: Affinity modification through antibody sequence mutation

The Panitumumab:EGFR complex structure (PDB: 5XS4) was analyzed, and the PISA program was used to calculate amino acids involved in antigen-antibody interactions. Amino acid residues on the Panitumumab CDR involved in binding EGFR were counted, wherein amino acid residues involved in providing hydrogen bonds, salt bridges and other interactions were defined as key amino acid residues, and the key amino acid residues were mutated to alanine to obtain antibodies with a reduced affinity, see Table 1 for details. Similarly, the Onartuzumab:c-MET complex structure (PDB: 4K3J) was analyzed, and the PISA program was used to calculate key amino acid residues involved in antigen-antibody interactions, and several antibodies with different affinities were designed according to the statistical results, see Table 2 for details.

**Table 1 List for EGFR mutant antibodies**

| Antibody Nos. | Mutation (or Wild Type) | | SEQ ID NOs: | |
|---|---|---|---|---|
| | VH | VL | VH | VL |
| Panitumumab | Wild Type | Wild Type | 18 | 19 |
| HM1 | D33A | Wild Type | 20 | 19 |
| HM2 | Y35A | Wild Type | 21 | 19 |
| HM3 | H52A | Wild Type | 22 | 19 |
| HM4 | Y55A | Wild Type | 23 | 19 |
| HM5 | N58A | Wild Type | 24 | 19 |
| HM7 | V102A | Wild Type | 25 | 19 |
| HM8 | T103A | Wild Type | 26 | 19 |
| LM1 | Wild Type | Y32A | 18 | 27 |
| LM3 | Wild Type | F91A | 18 | 28 |
| LM4 | Wild Type | D92A | 18 | 29 |
| LM5 | Wild Type | L96G | 18 | 30 |

**Table 2 List for c-MET mutant antibodies**

| Antibody Nos. | Mutation (or Wild Type) | | SEQ ID NOs: | |
|---|---|---|---|---|
| | VH | VL | VH | VL |
| Onartuzumab | Wild Type | Wild Type | 54 | 55 |
| OLM2 | Wild Type | A93Q | 54 | 56 |
| OLM4 | Wild Type | A93Y | 54 | 57 |
| OLM5 | Wild Type | K30Y | 54 | 58 |
| OLM6 | Wild Type | Q27G | 54 | 59 |
| OLM7 | Wild Type | T22S | 54 | 60 |

### Example 2: Preparation of antibodies

The common protein expression pcDNA3.4/CHO-K1 system are used for eukaryotic expression of antibodies, and expression plasmids were synthesized by Taizhou Biointron Co., Ltd.

250 ml of CHO cells were inoculated into 1L shake flasks at a density of 0.6×10⁶ cells/mL, and continued to be cultured until the cell density reached 3.5-4×10⁶ cells/mL. The cell culture was collected by centrifugation at 1000 rpm at room temperature for 5 min, the supernatant was discarded, the cells were resuspended in a freshly preheated medium, and placed in a cell shaker for transfection. 5 mL of medium was used to dilute 500 µg of target protein expression plasmid, and 5 mL of medium was used to dilute 3 mg of PEI. After mixing, they were left at room temperature for 5 min, and the plasmid/PEI mixture was directly added to the cells to be transfected. 4 hours after transfection, supplemented medium and feed were added to each bottle of cells. After 24 hours, sodium valproate was added to each bottle at a final concentration of 3.8 mM. After 72 hours, glucose was added to each bottle at a final concentration of 4 g/L. The culture supernatant was collected 5-6 days after transfection. The supernatant was purified using Protein A to obtain purified antibodies for subsequent detection.

### Example 3: Affinity activity of EGFR mutant antibodies to antigen-expressing cells

Flow cytometry was used to detect the binding of antibodies to target cells expressing antigens. Target cells in logarithmic growth phase were collected, washed with PBS, centrifuged, and resuspended in 2% FBS PBS. Each well was plated with 100 µL of 1×10⁵ cells, and centrifuged at 400 ×g for 2 minutes. Different concentrations of antibodies to be detected were added, incubated in a 4°C refrigerator for 1 hour, washed with 2% FBS PBS, and centrifuged at 400g for 2 min. 100 µL of secondary antibody with fluorescent groups was added, incubated in a 4°C refrigerator for 30 min, washed with 2% FBS PBS, and then detected on BD FACSLyric^{™}.

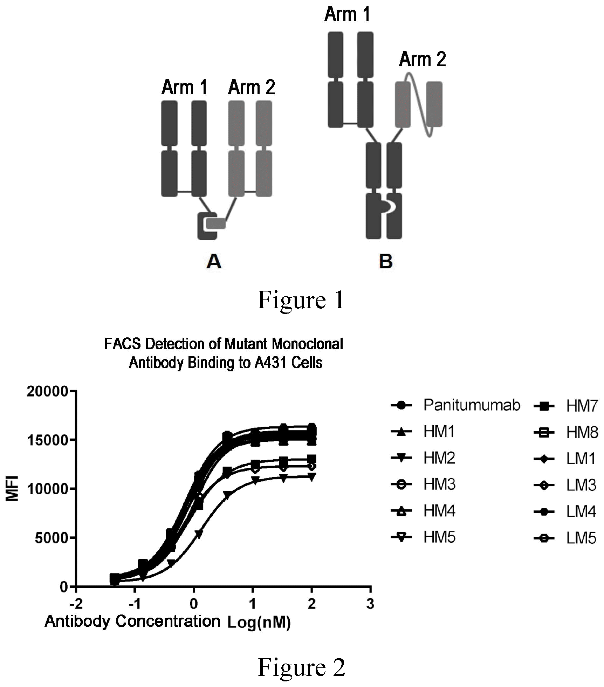

A431 cell line (purchased from ATCC, CRL-1555^{™}) was used in Figure 2, and the results showed that Eₘₐₓ activated by some mutant antibodies to EGFR antigen-expressing cell lines is lower than that of the parent antibody.

**Table 3 Affinity data of some mutant antibodies to A431 cells**

| Antibody Nos. | EC₅₀(nM) | Eₘₐₓ(nM) |
|---|---|---|
| Panitumumab | 0.84 | 15811 |
| HM2 | 1.35 | 11271 |
| HM7 | 0.83 | 13021 |
| LM3 | 0.70 | 12320 |

### Example 4: Detection of competitive binding activity of the mutant antibodies with parent antibodies

After site-directed mutagenesis, the affinity of EGFR antibodies changed, but the affinity epitopes of the antibodies to the antigen would not be affected. The competitive binding ability between the mutant antibodies could be detected by a flow cytometry.

A431 cells (purchased from ATCC, CRL-1555^{™}) in logarithmic growth phase were collected, washed with PBS, centrifuged, and resuspended in 2% FBS/PBS. Each well was plated with 100 µL of 1×10⁵ cells, and centrifuged at 400 ×g for 2 minutes. Cells were stimulated with different concentrations of the mutant antibodies combined with fixed concentrations of competitive antibodies, incubated in a 4°C refrigerator for 1 hour, washed with 2% FBS/PBS, and centrifuged at 400g for 2 min. 100 µL of PE anti-His Tag Antibody with fluorescent groups (purchased from biolegend, 362603) was added, incubated in a 4°C refrigerator for 30 min, washed with 2% FBS/PBS, and then detected on BD FACSLyric^{™}.

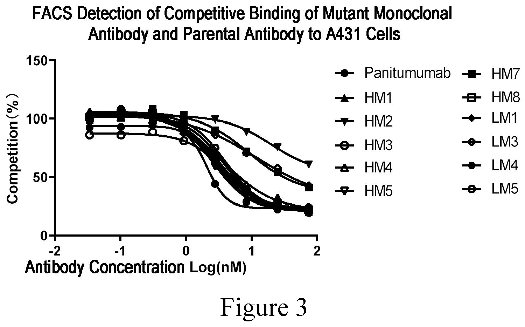

Figure 3 shows that the competitive binding activity of different site-directed mutant antibodies to the parent antibody was lower than that of the parent antibody, and the level of the competitive binding activity was consistent with the affinity level shown in Figure 2.

**Table 4 Competitive binding activity of the mutant antibodies to parent antibodies on A431 cells**

| Antibody Nos. | IC₅₀ | Antibody Nos. | IC₅₀ |
|---|---|---|---|
| Panitumumab | 2.09 | HM7 | 9.04 |
| HM1 | 3.80 | HM8 | 2.97 |
| HM2 | 20.11 | LM1 | 3.23 |
| HM3 | 3.93 | LM3 | 11.75 |

### Example 5: Expression of bispecific F(ab')₂ fragments in cell-free system

An appropriate amount of cell-free extract was thawed to room temperature, 50 mM iodoacetamide was added and incubated for 30 minutes. 8 mM magnesium glutamate, 10 mM ammonium glutamate, 130 mM potassium glutamate, 35 mM sodium pyruvate, 1.2 mM AMP, 0.86 mM each of GMP, UMP and CMP, 2 mM amino acids (tyrosine 1 mM), 4 mM sodium oxalate, 1 mM putrescine, 1.5 mM spermidine, 15 mM potassium phosphate, 100 nM T7 RNAP, 2-10 mg/mL plasmid DNA template, 10 mM GSH and 5 mM GSSG were added. The reaction was carried out at 180 rpm, 25°C for 16 hours, the supernatant was collected by centrifugation, and finally a F(ab')₂ protein was purified using an automated purification instrument, with reference to the method in Cell-free gene expression. Nat Rev Methods Primers 1, 49 (2021). The F(ab')₂ antibody numbers and the corresponding mutation numbers are shown in Table 3.

**Table 5 F(ab')₂ antibody combinations**

| Antibody Nos. | HM2 | HM3 | HM5 | Panitumumab | HM4 | HM7 | HM8 |
|---|---|---|---|---|---|---|---|
| Onartuzumab | A1 | B1 | C1 | D1 | E1 | F1 | G1 |
| OLM2 | A2 | B2 | C2 | D2 | E2 | F2 | G2 |
| OLM4 | A3 | B3 | C3 | D3 | E3 | F3 | G3 |
| OLM5 | A4 | B4 | C4 | D4 | E4 | F4 | G4 |
| OLM6 | A5 | B5 | C5 | D5 | E5 | F5 | G5 |
| OLM7 | A6 | B6 | C6 | D6 | E6 | F6 | G6 |

### Example 6: Detection of affinity activity of the bispecific F(ab')2 fragments to antigen-expressing cells

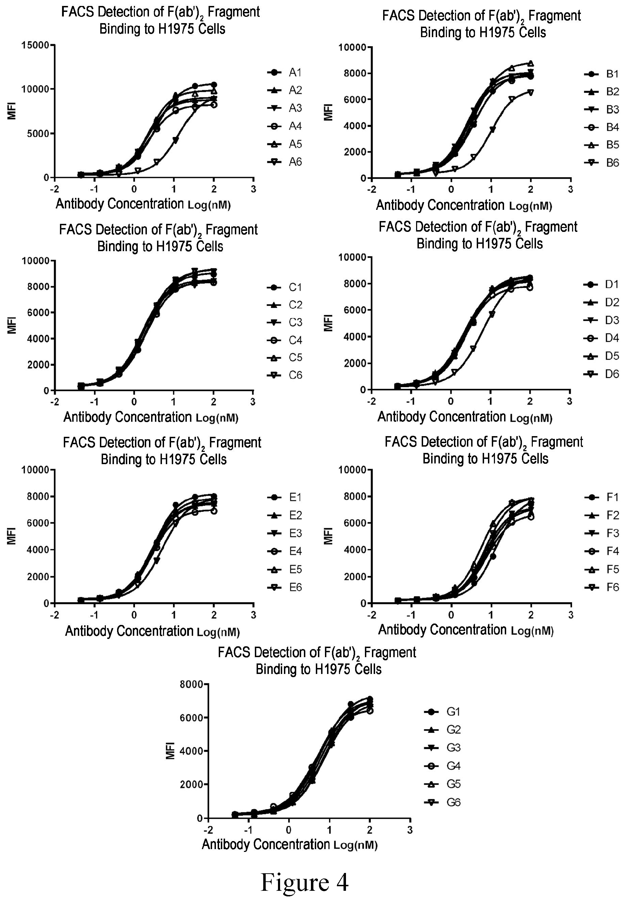

The method described in Example 3 was used to detect the affinity activity of the bispecific F(ab')₂ fragments to antigen-expressing cells, and the results are shown in Figures 4-6.

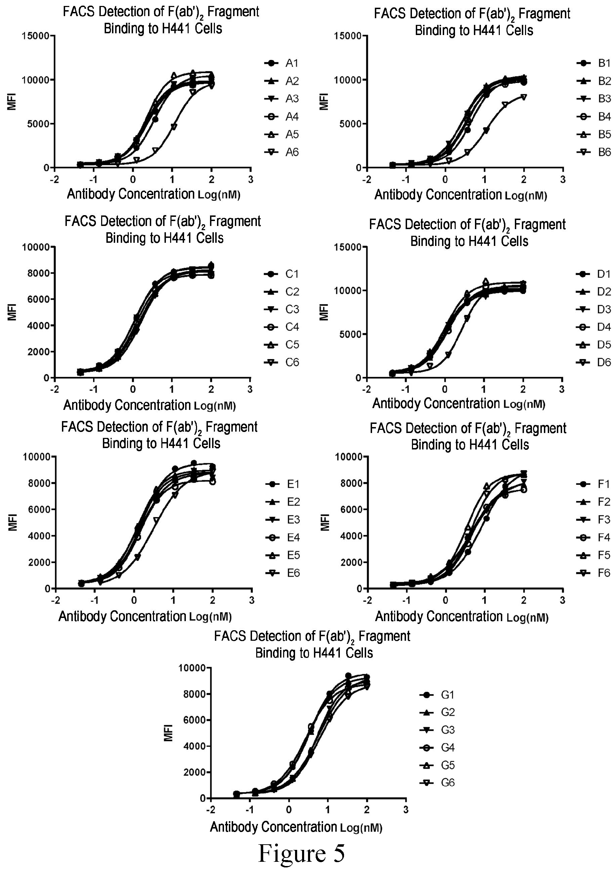

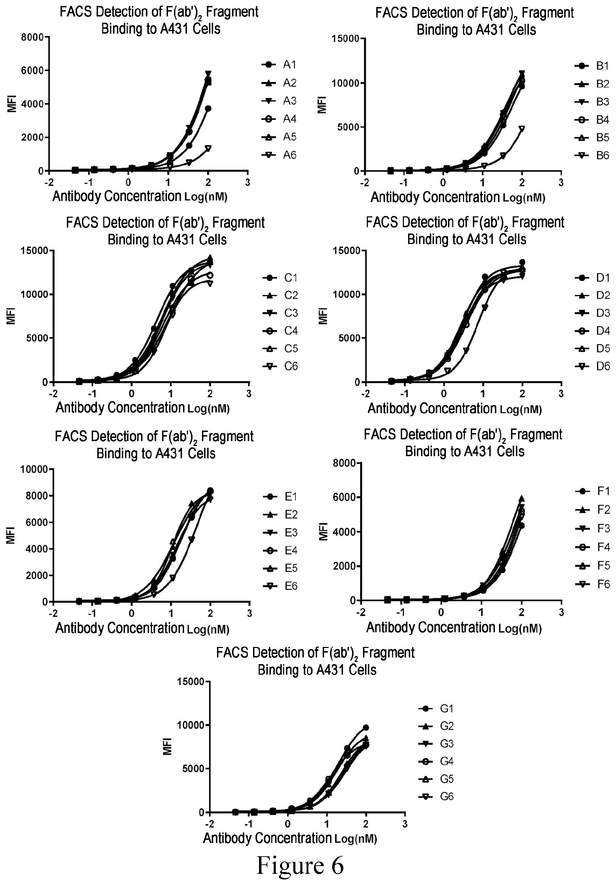

H1975 cell line (purchased from ATCC, CRL-5908) was used in Figure 4. The results showed that the affinity activities of F(ab')₂ composed of OLM7 with HM2, HM3, and Panitumumab were lower than those in the same group. H441 cell line (ATCC, CRM-HTB-174) was used in Figure 5. The results showed that the affinity activities of F(ab')₂ composed of OLM7 with HM2, HM3, and HM4 were lower than those in the same group. A431 cell line was used in Figure 6. The results showed that the affinity activities of groups A, B, F, and G were lower than those of the control group D. The fluorescent group-labeled secondary antibody PE anti-His Tag Antibody (purchased from biolegend, 362603) was used in all Figures 4-6.

### Example 7: Activity of the bispecific F(ab')₂ fragments simultaneously binding to two antigen epitopes

To develop bispecific antibodies simultaneously targeting two different antigens on a same cell, the bispecific antibodies require the ability to simultaneously bind to two different antigens. The enzyme-linked immunosorbent assay was used to detect the activity of the bispecific F(ab')₂ fragments simultaneously binding to EGFR and c-MET antigens.

Using the enzyme-linked immunosorbent assay, 1 µg/mL c-MET protein (purchased from Novoprotein, CS57) was coated on a 96-well plate. After serial dilution, the samples to be detected were added to the 96-well plate. After complete incubation, 0.25 µg/mL EGFR protein (purchased from SinoBiological, 10001-H27H-B) was added to bind to the other site of the antibody, followed by addition with 1:2000 diluted Peroxidase-Streptavidin (purchased from Jackson, AB_2337238) for incubation. Finally, TMB was used for color development. After the reaction was terminated, the measurement was performed at 450 nm, and the results were plotted and analyzed.

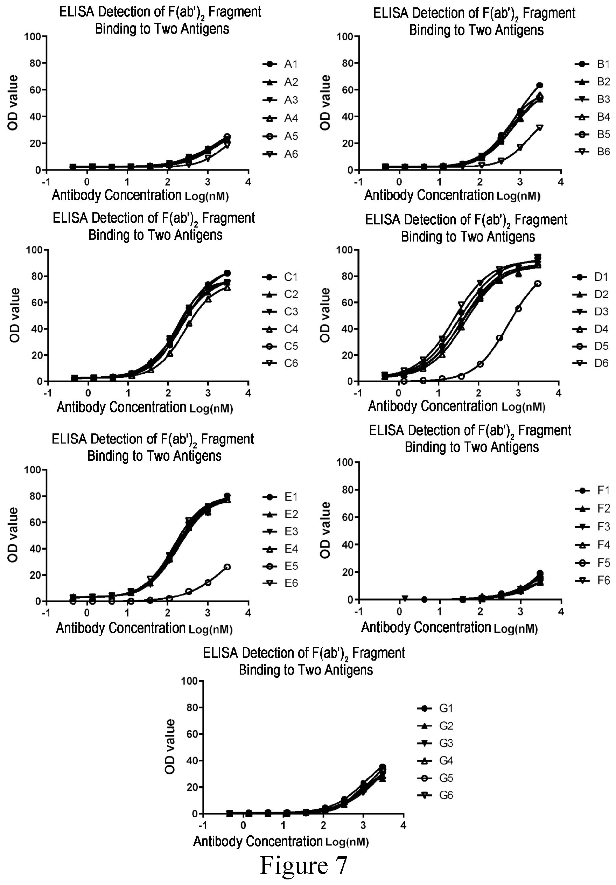

Results of Figure 7 showed that all antibodies in F(ab')₂ format possessed the ability to simultaneously bind to both antigens.

### Example 8: Detection of activity of the bispecific F(ab')₂ fragments mediating cell surface antigen internalization

The internalization activity of the antibodies can be detected by tracer labeling of the antibodies. CypHer5E is a red-excited fluorescent, pH-sensitive cyanine dye. It is non-fluorescent at alkaline pH and fluorescent at acidic pH, with a pKa of 7.3. CypHer5E mono NHS ester is suitable for labeling monoclonal antibodies and other proteins. CypHer5E-labeled secondary antibodies were used to detect the internalization of targets mediated by primary antibodies. When CypHer5E secondary antibodies enter acidic intracellular endosomes from the neutral cell surface, the internalization activity in a time-dependent manner can be reflected by detecting the strength of the fluorescent signal.

The Incucyte instrument (purchased from Sartorius) was used to detect the internalization effect of the antibodies on cells. Cells in logarithmic growth phase were collected, washed with PBS, centrifuged, and resuspended in 10% FBS DMEM medium. Each well was plated with 80 µL of 1×10⁴ cells and placed in a 37°C±2°C, 5% CO₂ constant temperature incubator for 21 h. Preparation of dye: AffiniPure Fab Fragment Goat Anti-Human IgG (purchased from Jackson, 109-007-008) was diluted to 1 mg/mL, and the required CypHer5E Mono NHS Ester (purchased from Cytiva, PA15405) was calculated according to the molar ratio of secondary antibody:dye = 1:20 and added, then incubated at room temperature protected from light for 1 h. After dialysis using Slide-A-Lyzer^{™} Mini Dialysis Cups (purchased from Thermo Scientific, 88404), the concentration was detected. The first well of the dilution plate was added according to the molar ratio of primary antibody:dye-labeled secondary antibody = 1:3, then placed in a 37°C incubator for 30 minutes to form complexes. The complexes were then gradient diluted and added to the cell plate according to concentration layout, 20 µL/well, placed in the Incucyte for culture and detection of cell fluorescence intensity at 24 h.

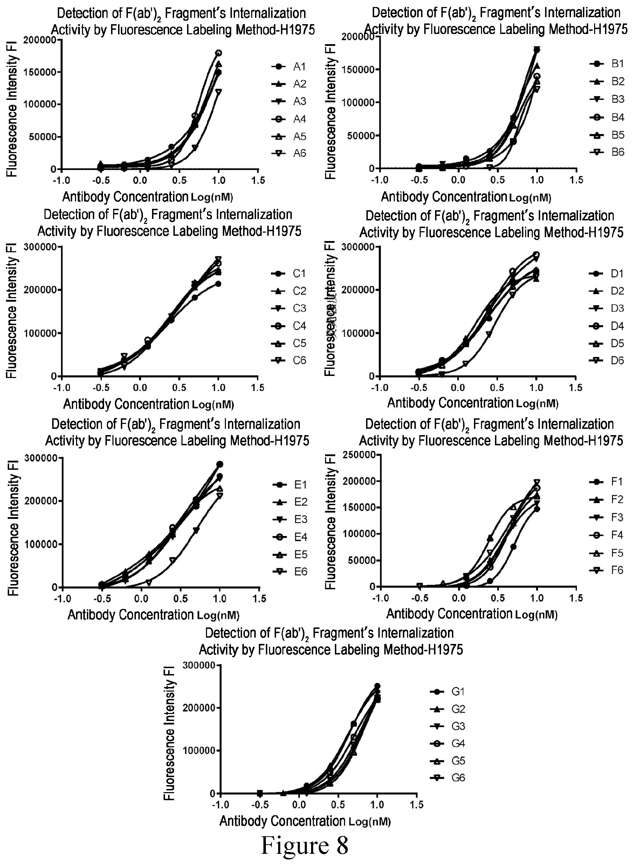

H1975 cell line was used in Figure 8. The results showed that the maximum internalization activity of the antibodies of group C and group E was comparable to that of the wild-type antibody of group D, while groups A, B, and F were weaker than the control group D.

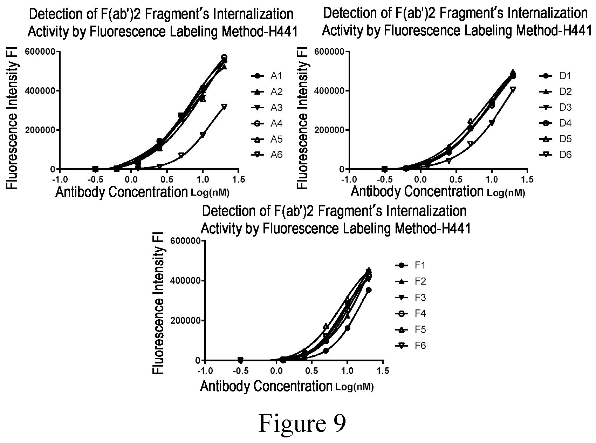

H441 cell line was used in Figure 9. The results showed that group A exhibited a superior internalization activity.

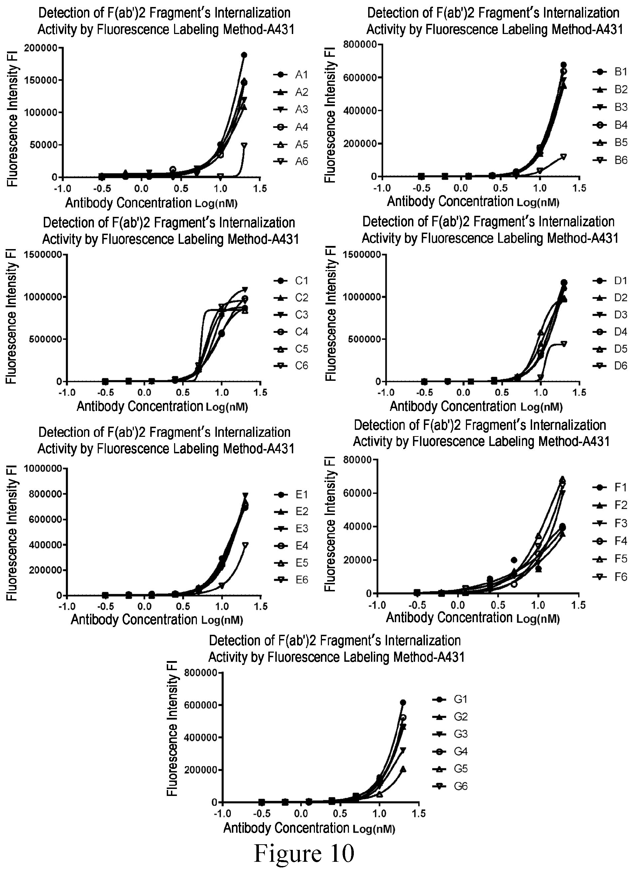

A431 cell line was used in Figure 10. The results showed that the maximum internalization activity of groups A and F was much lower than that of the wild-type group. The EGFR protein expression level of the A431 cell line was much higher than that of c-MET protein, indicating that the corresponding EGFR mutations in groups A and F had potential safety advantages.

### Example 9: Expression of the bispecific antibodies in eukaryotic expression system

The common protein expression pcDNA3.4/CHO-K1 system was used for eukaryotic expression of the bispecific antibodies. Expression plasmids were synthesized by Taizhou Biointron Co., Ltd. Bispecific antibodies for subsequent detection were prepared using the method described in Example 2. The antibody numbers and corresponding mutation sites are shown in the table below.

**Table 6 List for eukaryotic expression of the bispecific antibodies**

| | Arm 1:c-MET | | SEQ ID NOs: | | Arm 2:EGFR | | SEQ ID NOs: | |
|---|---|---|---|---|---|---|---|---|
| Antibody Nos. | Mutant (or Wild Type) | | | | Mutant (or Wild Type) | | | |
| | VH | VL | VH | VL | VH | VL | VH | VL |
| SVAB14 | Wild Type | Wild Type | 54 | 55 | Wild Type | Wild Type | 18 | 19 |
| SVAB38 | Wild Type | Wild Type | 54 | 55 | Y35A | Wild Type | 21 | 19 |
| SVAB39 | Wild Type | Wild Type | 54 | 55 | Wild Type | F91A | 18 | 28 |
| SVAB42 | Wild Type | Wild Type | 54 | 55 | D33A | F91A | 20 | 28 |
| SVAB43 | Wild Type | Wild Type | 54 | 55 | Y55A | Wild Type | 23 | 19 |
| SVAB44 | Wild Type | Wild Type | 54 | 55 | V102A | Wild Type | 25 | 19 |

### Example 10: Affinity activity of the bispecific antibodies to antigen-expressing cells

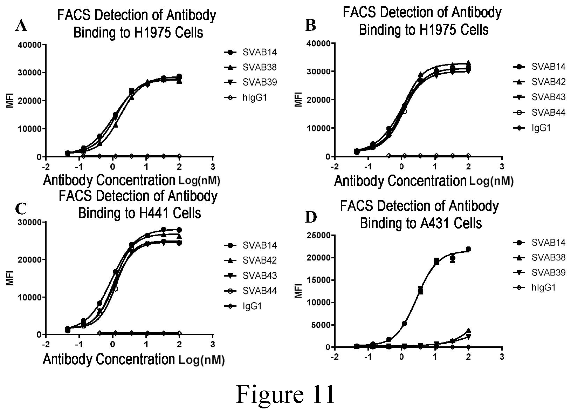

The method described in Example 3 was used to detect the affinity activity of the bispecific antibodies to antigen-expressing cells, and the results are shown in Figure 11.

H1975 cell line was used in Figures 11A-B, H441 cell line was used in Figure 11C, and A431 cell line was used in Figure 11D. The same fluorescent group-labeled secondary antibody (purchased from Invitrogen, 12-4998-82) was used in Figure 11. The cell line affinity activity results in Figure 11 showed that SVAB38 and SVAB39 had a strong affinity for H1975 cells, close to the activity of wild-type SVAB14, but had a low affinity on the A431 cell line.

**Table 7 Comparison of EC₅₀ affinity of the bispecific antibodies to H1975, H441 and A431 cells**

| Antibody Nos. | H1975-EC₅₀(nM) | H441-EC₅₀(nM) | A431-EC₅₀(nM) |
|---|---|---|---|
| SVAB14 | 0.95 | 0.93 | 2.85 |
| SVAB38 | 1.67 | N.D. | >50 |
| SVAB39 | 1.23 | N.D. | >50 |
| SVAB42 | 1.10 | 1.14 | N.D. |
| SVAB43 | 1.06 | 1.11 | N.D. |
| SVAB44 | 1.20 | 1.26 | N.D. |

### Example 11: Detection of cell surface antigen reduction mediated by the bispecific antibodies

The internalization activity of antibodies can be detected either by tracer labeling of the antibodies or by the cell killing effect of toxins carried by the antibodies. Different internalization activity detection schemes were used in this example to detect the internalization mediated by the bispecific antibodies.

### A) Fluorescence Internalization Method

The same fluorescence internalization method as in Example 8 was used to detect the cell surface antigen reduction mediated by the bispecific antibodies.

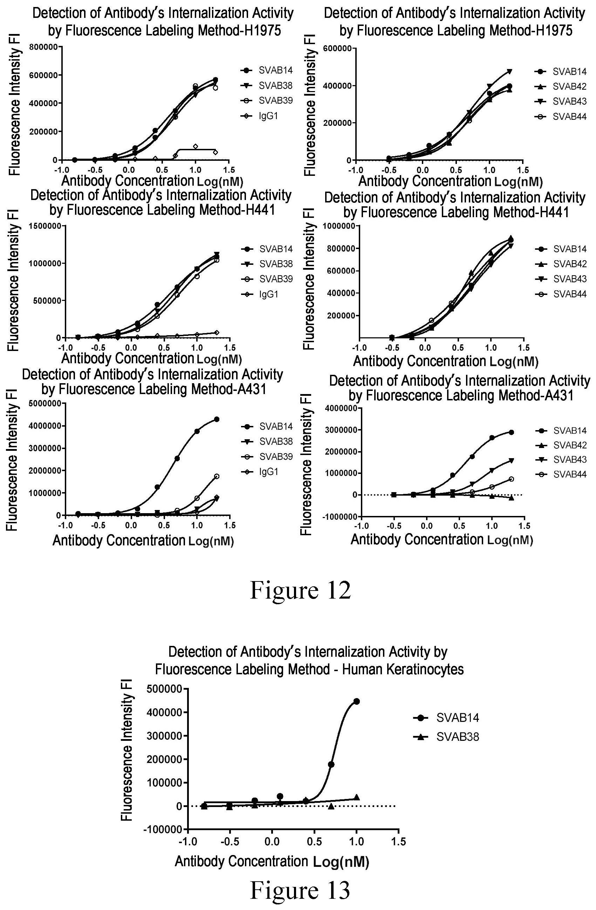

Results of Figure 12 showed that in H1975 and H441 cell lines, the internalization activities of the bispecific antibodies that are synthesized with different mutated EGFR antibody sequences were similar, while the results in the A431 cell line showed that internalization activity was related to affinity, and lower EGFR affinity could improve potential safety.

Human primary epidermal keratinocytes HEKs (ATCC, PCS-200-011) was used in Figure 13. The results showed that the antibody SVAB38 with a reduced EGFR affinity had a significantly reduced internalization activity on this cell compared to the EGFR wild-type bispecific antibody SVAB14.

### B) Toxin Secondary Antibody Method

Monomethyl auristatin E (MMAE) is a cytotoxin that can kill cancer cells.

MMAE is an analog of Dolastatin 10. Microtubules are composed of α-tubulin and β-tubulin and are the main components of the cytoskeleton. Tubulin inhibitors kill tumor cells by disrupting the cytoskeletal structure of tumor cells and interfering with mitosis. MMAE toxin-labeled secondary antibodies can enter cells and kill tumor cells by binding to primary antibody molecules with internalization activity. The killing effect is evaluated by detecting the amount of remaining viable cells. Therefore, MMAE secondary antibodies are also commonly used to detect the ability of primary antibody molecules to cause cell membrane antigen internalization.

Cell proliferation inhibition was used to detect the activity of antibody-mediated cell surface antigen internalization. Cells were inoculated in 96-well cell plates. After cell adhesion, MMAE-labeled anti-Fc secondary antibody (purchased from MORADEC, AH-202AE) was pre-incubated with the samples to be detected at a molar ratio of 6:1 at 37°C for 30 min, then serially diluted and added to the cell culture plate for incubation for a period of time. CTG was added to detect fluorescence, and the cell killing rate was calculated based on fluorescence intensity.

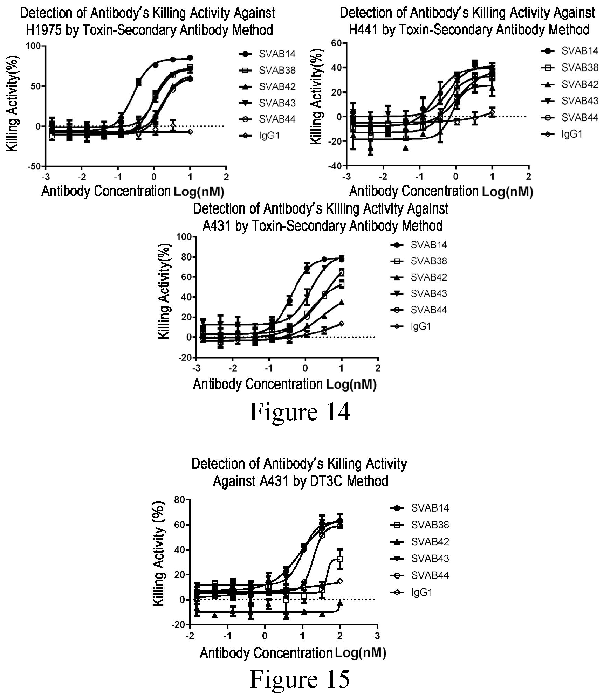

Results of Figure 14 showed that SVAB14, the bispecific antibody of wild-type sequences combination, mediated the lowest EC₅₀ internalization activity on H1975 and H441 cells, but also showed the lowest EC₅₀ internalization activity on the A431 cell line, suggesting that the stronges*t in vitro* efficacy might also lead to stronger safety risks. In contrast, the SVAB38 molecule showed an internalization activity slightly weaker than the wild type on 1975 and H441 cells, but a significantly reduced internalization on the A431 cell line.

### C) DT3C Method

DT3C is a recombinant protein produced by genetic recombination technology. The protein includes a diphtheria toxin (DT) without a receptor-binding domain and the C1, C2, C3 (3C) domains of Streptococcus protein G. After the mAb-DT3C conjugate recognizes and binds to the cell surface antigen, the mAb-DT3C-antigen complex is internalized; then DT3C is cleaved by cytoplasmic furin protease, and the catalytic domain of DT3C is released into the cytoplasm. The catalytic domain of DT3C causes ADP-ribosylation of elongation factor (EF)-2, subsequently leading to cytotoxicity by inhibiting the protein translation mechanism.

Cell proliferation inhibition was used to detect the activity of cell surface antigen internalization mediated by the antibodies. Cells were inoculated in 96-well cell plates. After cell adhesion, DT3C (purchased from Huamei Bio, CSB-EP360556CQR1) was pre-incubated with the samples to be detected at a molar ratio of 4:1 at 37°C for 30 min, then serially diluted and added to the cell culture plate for incubation for a period of time. CTG was added to detect fluorescence, and the cell killing rate was calculated based on fluorescence intensity.

Results of Figure 15 showed that in the A431 cell line with a high EGFR expression, the internalization activity of SVAB38 was significantly lower than that of the wild-type SVAB14 bispecific antibody molecule, suggesting potential safety advantages.

### Example 12: Detection of cell surface antigen reduction mediated by the antibodies

The binding of antibodies to specific surface antigen targets mediates surface antigen internalization, which is further degraded through the lysosomal pathway. To detect the activity of the antigen reduction mediated by the antibodies, the ELISA method was used to detect amount of the antigen. The specific experimental methods are as follows:
Sample preparation: 2×10⁵ cells/well of A549 cells (purchased from ATCC, CCL-185) were inoculated in 6-well cell plates, cultured overnight, and then 30 µg/mL of antibody samples were added and placed in the incubator overnight. Cell lysis buffer was added, and the supernatant was collected by centrifugation.

EGFR protein detection: 0.25 µg/mL Panitumumab (purchased from Biointron, B21796801) was coated on a 96-well plate. 100 ng/mL of EGFR protein (purchased from Kactus Biosystems, EGF-HM201) was gradient diluted and added to the 96-well plate together with several-fold diluted samples to be detected. After complete incubation, 0.25 µg/mL EGFR-biotin antibody (purchased from SinoBiological, 10001-MM08T-B) was added, followed by addition with 1:2000 diluted Peroxidase-Streptavidin (purchased from Jackson, AB_2337238) for incubation. Finally, TMB was used for color development. After the reaction was terminated, the measurement was performed at 450 nm. A standard curve was plotted for the EGFR protein group with a good linear relationship on the slope, and an equation was obtained. The OD value of the sample group was substituted to calculate the concentration.

c-MET detection: 2 µg/mL c-MET antibody (purchased from Sino Biological, 10692-R261) was coated on a 96-well plate. 100 ng/mL of c-MET protein (purchased from Novoprotein, CS57) was gradient diluted and added to the 96-well plate together with several-fold diluted samples to be detected. After complete incubation, 1 µg/mL c-MET antibody was added, followed by addition with 1:5000 diluted Fab-HRP (purchased from GenScript, C5592HI060-4/P9HI001) for incubation. Finally, TMB was used for color development. After the reaction was terminated, the measurement was performed at 450 nm. A standard curve was plotted for the c-MET protein group with a good linear relationship on the slope, and an equation was obtained. The OD value of the sample group was substituted to calculate the concentration.

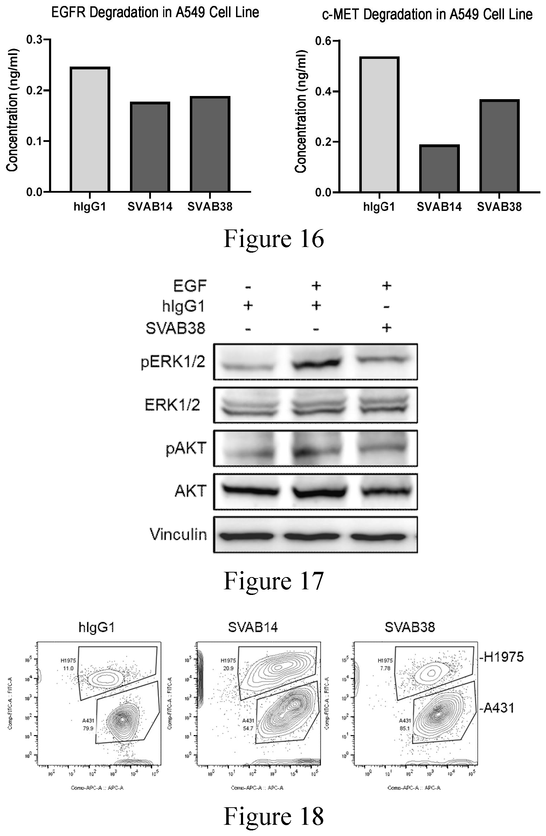

Results of Figure 16 showed that compared with the negative control antibody, the SVAB38 molecule had an activity in promoting degradation of the EGFR and c-MET antigens.

### Example 13: Detection of inhibitory activity of the antibodies on EGF-activated target cell downstream signals

Epidermal growth factor (EGF) is one of the main ligands of EGFR currently discovered. Due to the binding of EGF ligand to the extracellular receptor, EGFR dimerization causes intracellular domain to undergo tyrosine kinase activation, that is, the intracellular TK domain undergoes biochemical reaction with ATP, ATP becomes ADP, and TK phosphorylation activates EGFR enzyme activity. Activated EGFR can transmit proliferation signals and anti-apoptotic signals to the nucleus through multiple downstream signal transduction pathways such as PI3K-AKT-mTOR, Ras-Raf-MEK-ERK1/2, etc., controlling cell growth and division. Antibodies can inhibit EGF activation of downstream signals by competitively binding to the extracellular EGF binding epitope of EGFR. The activation signals are commonly expressed by the phosphorylation levels of AKT and ERK.

Western blot was used to detect target cell downstream signal pathways. H1975 cells were plated in 6-well plates at a density of 4×10⁵ cells/well. After cell adhesion, the medium was changed to serum-free medium for overnight culture, then pretreated with 30 µg/mL test antibody for 1 h, followed by addition with 100 ng/mL EGF (purchased from SinoBiological, 10605-H01H) for stimulation for 15 min. The medium was removed, cells were washed once with pre-cooled PBS, and 200 µL of lysis buffer (purchased from Thermo Fisher, FNN0011) containing protease inhibitor (purchased from Thermo, 78425) and phosphatase inhibitor (purchased from Roche, 04906845001) was added, treated on ice for 30 min, and cell lysate was collected with a cell scraper. After centrifugation at 13,000 rpm for 10 min at 4°C, the supernatant was transferred to a new 1.5 mL EP tube for further use. The protein concentration in the protein supernatant was determined using a BCA kit (purchased from Beyotime, P0010S), and then 2× SDS-page loading buffer was added and the protein was heated in a metal bath for 5 minutes. After electrophoresis with 8% precast gel (purchased from GenScript, M00657), it was transferred to PVDF membrane (purchased from Thermo, IB31002). The PVDF membrane was blocked with 5% BSA-TBST blocking solution for 1 h, then blocked overnight at 4°C with primary antibodies (purchased from CST, anti-AKT-rabbit 9272, Anti-P-AKT(Ser473)-rabbit 9271, p44/42 MAPK (Erk1/2) antibody 9102, Phospho-p44/42 MAPK (Erk1/2) (Thr202/Tyr204) Antibody 9101, Vinculin Rabbit mAb 13901). After washing 3 times with TBST, 5 min each time, the PVDF membrane was incubated with HRP-conjugated secondary antibody (purchased from GenScript, A01827) at room temperature for 1 h, and washed 3 times with TBST, 5 min each time. Finally, developing solution (purchased from Merck Millipore, WBKLS0500) was added for color development.

Results of Figure 17 showed that compared with the negative control antibody, SVAB38 could significantly reduce the phosphorylation levels of downstream AKT and ERK.

### Example 14: Detection of selective killing of different antibodies on keratinocytes

The main common adverse reactions of EGFR-targeted drugs are skin adverse reactions, including rash, dry skin, and paronychia, etc. EGFR is not only a tumor target, but also widely expressed in the human body, especially highly expressed in epithelial cells of tissues related to these skin side effects. In order to detect whether antibodies more specifically target tumor tissue rather than normal tissue, the A431 cell line with antigen expression similar to tissue epithelial cells was selected as a substitute for normal cells, and H1975 cells were selected as tumor target cells.

A431 and H1975 cells in logarithmic growth phase were collected, washed with PBS, centrifuged, and resuspended in a complete medium. According to the instructions, A431 cells were labeled with CellTrace^{™} Far Red Cell Proliferation Kit (Thermo, C34564-180T); and H1975 cells were labeled with CellTrace^{™} CFSE Cell Proliferation Kit (Thermo, C34554-180T). Then, the cells were mixed at a 1:1 ratio according to cell density, and each well was plated with 1900 µL of 2×10⁵ cells, and placed in a 37°C±2°C, 5% CO₂ constant temperature incubator for overnight culture. The next day, 100 µL of different concentrations of antibodies and toxin secondary antibodies were added to treat the cells. After 3-5 days of treatment, the cells were collected, washed with PBS, centrifuged, resuspended in 2% FBS/PBS, and then detected on BD FACSLyric^{™}.

Flow cytometry results of Figure 18 showed that compared with the wild-type control antibody SVAB14, the SVAB38 antibody had an obvious killing selectivity for killing H1975 cells.

## Claims

1. An antibody or antigen-binding fragment thereof specifically binding to human epidermal growth factor receptor (hEGFR), wherein the antibody or antigen-binding fragment comprises:
(a1) a heavy chain variable region (VH), comprising HCDR1, HCDR2 and HCDR3, which according to the Kabat sequence numbering system respectively comprise the amino acid sequences shown in SEQ ID NO: 7, SEQ ID NO: 2 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a2) a heavy chain variable region (VH), comprising HCDR1, HCDR2 and HCDR3, which according to the Kabat sequence numbering system respectively comprise the amino acid sequences shown in SEQ ID NO: 8, SEQ ID NO: 2 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a3) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 9 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a4) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 10 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a5) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 11 and SEQ ID NO: 3 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a6) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 12 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a7) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 13 or variants thereof, and (b1) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 6 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b2) a light chain variable region (VL), comprising LCDR1, LCDR2 and LCDR3, which according to the Kabat sequence numbering system respectively comprise the amino acid sequences shown in SEQ ID NO: 14, SEQ ID NO: 5 and SEQ ID NO: 6 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b3) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 15 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b4) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 16 or variants thereof; or
(a1) a heavy chain variable region (VH), comprising HCDR1, HCDR2 and HCDR3, which according to the Kabat sequence numbering system respectively comprise the amino acid sequences shown in SEQ ID NO: 1, SEQ ID NO: 2 and SEQ ID NO: 3 or variants thereof, and (b5) a light chain variable region (VL), comprising LCDR1, LCDR2 and LCDR3, which according to the Kabat sequence numbering system respectively comprise the amino acid sequences shown in SEQ ID NO: 4, SEQ ID NO: 5 and SEQ ID NO: 17 or variants thereof;
wherein the variants of CDRs have 3, 2 or 1 amino acid differences from, or have at least 80%, 85%, 90%, 91%, 92%, 93%, 94%, 95%, 96%, 97%, 98%, 99% identity to the corresponding CDRs, respectively.

2. The antibody or antigen-binding fragment thereof according to claim 1, wherein the VH and the VL are selected from the group consisting of, or variants thereof:
(a) a heavy chain having a variable domain comprising an amino acid sequence selected from any one of SEQ ID NOs: 20-26 and a light chain having a variable domain comprising the amino acid sequence shown in SEQ ID NO: 19; or
(b) a heavy chain having a variable domain comprising the amino acid sequence shown in SEQ ID NO: 18 and a light chain having a variable domain comprising an amino acid sequence selected from any one of SEQ ID NOs: 27-30;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

3. The antibody or antigen-binding fragment thereof according to claim 1 or 2, wherein the antibody comprises a monoclonal antibody, a polyclonal antibody, a multispecific antibody, a chimeric antibody, a humanized antibody or a fully human antibody; and/or, the antigen-binding fragment comprises an Fab, an Fab', an Fv fragment, an F(ab')₂, an Fd fragment, a dAb, a complementarity determining region fragment, an scFv or a single-domain antibody.

4. The antibody or antigen-binding fragment thereof according to claim 1 or 2, wherein the antigen-binding fragment is an scFv, sequentially comprising a light chain variable region, a linking fragment and a heavy chain variable region from N-terminus to C-terminus.

5. The antibody or antigen-binding fragment thereof according to claim 4, wherein the linking fragment is selected from (GS)n, (GGS)n, (GGGS)n, or (GGGGS)n, wherein n is selected from 2, 3, or 4.

6. The antibody or antigen-binding fragment thereof according to claim 5, wherein the linking fragment comprises the amino acid sequence shown in SEQ ID NO: 78.

7. The antibody or antigen-binding fragment thereof according to claim 1 or 2, wherein the antigen-binding fragment is an scFv, comprising any one of the amino acid sequences shown in SEQ ID NOs: 32-42 or variants thereof, wherein the variants have 3, 2 or 1 amino acid differences from, or have at least 80%, 85%, 90%, 91%, 92%, 93%, 94%, 95%, 96%, 97%, 98%, 99% identity to said scFv, respectively.

8. The antibody or antigen-binding fragment thereof according to claim 7, wherein the scFv further comprises point mutations H44C and L100C.

9. The antibody or antigen-binding fragment thereof according to any one of claims 1-8, wherein a maximum effect induced by the binding of the antibody or antigen-binding fragment to an EGFR-expressing cell line is more than 10% lower than a maximum effect induced by the binding of the parent antibody to said EGFR-expressing cell line.

10. The antibody or antigen-binding fragment thereof according to any one of claims 1-8, wherein the antibody or antigen-binding fragment inhibits Panitumumab binding to an EGFR-expressing cell line with an IC₅₀ value higher than 2.10 nM.

11. A multispecific antibody, comprising a plurality of different antigen-binding domains, wherein a first antigen-binding domain comprises the antibody or antigen-binding fragment thereof according to any one of claims 1-10.

12. A bispecific antibody, comprising two different antigen-binding domains, wherein a first antigen-binding domain comprises the antibody or antigen-binding fragment thereof according to any one of claims 1-10, and a second antigen-binding domain comprises an antibody or antigen-binding fragment thereof specifically binding to a tumor-associated antigen (TAA).

13. The bispecific antibody according to claim 12, wherein the tumor-associated antigen is selected from any one of the group consisting of c-MET, AFP, ALK, BAGE protein, BIRC5, BIRC7, β-catenin, brc-abl, BRCA1, BORIS, CA9, carbonic anhydrase IX, caspase-8, CALR, CCR5, CD19, CD20, CD22, CD30, CD40, CDK4, CEA, CTLA4, cyclin-B1, CYP1B1, EGFRvIII, ErbB3, ErbB4, ETV6-AML, EpCAM, EphA2, Fra-1, FOLR1, GAGE protein, GD2, GD3, GloboH, glypican-3, GM3, gp100, Her2, HLA/B-raf, HLA/k-ras, HLA/MAGE-A3, hTERT, LMP2, MAGE protein, MART-1, mesothelin, ML-IAP, Muc1, Muc2, Muc3, Muc4, Muc5, Muc16, MUM1, NA17, NY-BR1, NY-BR62, NY-BR85, NY-ESO1, OX40, p15, p53, PAP, PAX3, PAX5, PCTA-1, PLAC1, PRLR, PRAME, PSMA, RAGE protein, Ras, RGS5, Rho, SART-1, SART-3, STEAP1, STEAP2, TAG-72, TGF-β, TMPRSS2, Thomson-Friedenreich antigen, TRP-1, TRP-2, tyrosinase and uroplakin-3.

14. The bispecific antibody according to claim 12, wherein the second antigen-binding domain specifically binds to c-MET.

15. The bispecific antibody according to claim 14, wherein the second antigen-binding domain specifically binding to c-MET comprises:
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b1) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b2) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 49 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b3) a light chain variable region (VL), comprising LCDR1 region, LCDR2 region and LCDR3 region, which according to the Kabat sequence numbering system respectively comprise the amino acid sequences shown in SEQ ID NO: 46, SEQ ID NO: 47 and SEQ ID NO: 50 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b4) a light chain variable region (VL), comprising LCDR1 region, LCDR2 region and LCDR3 region, which according to the Kabat sequence numbering system respectively comprise the amino acid sequences shown in SEQ ID NO: 51, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b5) a light chain variable region (VL), comprising LCDR1 region, LCDR2 region and LCDR3 region, which according to the Kabat sequence numbering system respectively comprise the amino acid sequences shown in SEQ ID NO: 52, SEQ ID NO: 47 and SEQ ID NO: 48 or variants thereof; or
(a1) a heavy chain variable region (VH), comprising HCDR1 region, HCDR2 region and HCDR3 region, which according to the Kabat sequence numbering system respectively comprise the amino acid sequences shown in SEQ ID NO: 43, SEQ ID NO: 44 and SEQ ID NO: 45 or variants thereof, and (b6) a light chain variable region (VL), comprising LCDR1 region, LCDR2 region and LCDR3 region, which according to the Kabat sequence numbering system respectively comprise the amino acid sequences shown in SEQ ID NO: 53, SEQ ID NO: 47 and SEQ ID NO: 48 or variants thereof;
wherein the variants of CDRs have 3, 2 or 1 amino acid differences from, or have at least 80%, 85%, 90%, 91%, 92%, 93%, 94%, 95%, 96%, 97%, 98%, 99% identity to the corresponding CDRs, respectively.

16. The bispecific antibody according to claim 14, wherein the second antigen-binding domain specifically binding to c-MET has:
1) a heavy chain comprising a variable domain having the amino acid sequence shown in SEQ ID NO: 54 or variants thereof; and
2) a light chain comprising a variable domain having an amino acid sequence selected from any one of SEQ ID NOs: 55-60 or variants thereof;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

17. The bispecific antibody according to any one of claims 14-16, wherein the second antigen-binding domain further comprises a first heavy chain constant region (CH1) and a light chain constant region (CL).

18. The bispecific antibody according to claim 17, wherein the CH1 comprises the amino acid sequence shown in SEQ ID NO: 75, and the CL comprises the amino acid sequence shown in SEQ ID NO: 76 or 77.

19. The bispecific antibody according to any one of claims 14-16, wherein the second antigen-binding domain consists of a light chain-heavy chain pair, wherein the light chain comprises any one of the amino acid sequences shown in SEQ ID NOs: 63-67, and the heavy chain comprises the amino acid sequence shown in SEQ ID NO: 61.

20. The bispecific antibody according to any one of claims 14-19, wherein the second antigen-binding domain comprises an Fab, an Fab', an Fv fragment, an F(ab')₂, an Fd fragment, a dAb, a complementarity determining region fragment, an scFv or a single-domain antibody.

21. The bispecific antibody according to claim 12, wherein the bispecific antibody further comprises an Fc domain.

22. The bispecific antibody according to claim 21, wherein the Fc domain is selected from an IgA Fc fragment, an IgG Fc fragment, an IgM Fc fragment, an IgD Fc fragment, or an IgE Fc fragment.

23. The bispecific antibody according to claim 22, wherein the Fc domain is selected from an IgG1 Fc fragment, an IgG2 Fc fragment, an IgG3 Fc fragment, or an IgG4 Fc fragment.

24. The bispecific antibody according to claim 22, wherein the Fc domain is an IgG1 Fc fragment comprising the amino acid sequence shown in SEQ ID NO: 79.

25. The bispecific antibody according to any one of claims 21-24, wherein the Fc domain further comprises amino acid substitutions that promote pairing of a first subunit and a second subunit of said Fc domain.

26. The bispecific antibody according to claim 25, wherein the first subunit of said Fc domain comprises amino acid substitutions:
Y349C/T366S/L368A/Y407V, and the second subunit of said Fc domain comprises amino acid substitutions: S354C/T366W.

27. The bispecific antibody according to any one of claims 12-26, selected from the group consisting of, or variants thereof:
(a) a first antigen-binding domain comprising HCDR1-3 with the sequences shown in SEQ ID NOs: 1-3 and LCDR1-3 with the sequences shown in SEQ ID NOs: 4-6; or
(b) a first antigen-binding domain comprising HCDR1-3 with the sequences shown in SEQ ID NOs: 8, 2 and 3 and LCDR1-3 with the sequences shown in SEQ ID NOs: 4-6; or
(c) a first antigen-binding domain comprising HCDR1-3 with the sequences shown in SEQ ID NOs: 1-3 and LCDR1-3 with the sequences shown in SEQ ID NOs: 4, 5, and 15; or
(d) a first antigen-binding domain comprising HCDR1-3 with the sequences shown in SEQ ID NOs: 7, 2, and 3 and LCDR1-3 with the sequences shown in SEQ ID NOs: 4, 5, and 15; or
(e) a first antigen-binding domain comprising HCDR1-3 with the sequences shown in SEQ ID NOs: 1, 10 and 3 and LCDR1-3 with the sequences shown in SEQ ID NOs: 4-6; or
(f) a first antigen-binding domain comprising HCDR1-3 with the sequences shown in SEQ ID NOs: 1, 2 and 12 and LCDR1-3 with the sequences shown in SEQ ID NOs: 4-6;
and a second antigen-binding domain comprising HCDR1-3 with the sequences shown in SEQ ID NOs: 43-45 and LCDR1-3 with the sequences shown in SEQ ID NOs: 46-48;
wherein the variants have 3, 2 or 1 amino acid differences from, or have at least 80%, 85%, 90%, 91%, 92%, 93%, 94%, 95%, 96%, 97%, 98%, 99% identity to the corresponding CDRs, respectively.

28. The bispecific antibody according to any one of claims 12-26, selected from the group consisting of, or variants thereof:
(a) a first antigen-binding domain comprising VH with the sequence shown in SEQ ID NO: 18 and VL with the sequence shown in SEQ ID NO: 19; or
(b) a first antigen-binding domain comprising VH with the sequence shown in SEQ ID NO: 21 and VL with the sequence shown in SEQ ID NO: 19; or
(c) a first antigen-binding domain comprising VH with the sequence shown in SEQ ID NO: 18 and VL with the sequence shown in SEQ ID NO: 28; or
(d) a first antigen-binding domain comprising VH with the sequence shown in SEQ ID NO: 20 and VL with the sequence shown in SEQ ID NO: 28; or
(e) a first antigen-binding domain comprising VH with the sequence shown in SEQ ID NO: 23 and VL with the sequence shown in SEQ ID NO: 19; or
(f) a first antigen-binding domain comprising VH with the sequence shown in SEQ ID NO: 25 and VL with the sequence shown in SEQ ID NO: 19;
and a second antigen-binding domain comprising VH with the sequence shown in SEQ ID NO: 54 and VL with the sequence shown in SEQ ID NO: 55;
wherein the variants have 3, 2 or 1 amino acid differences from, or have at least 80%, 85%, 90%, 91%, 92%, 93%, 94%, 95%, 96%, 97%, 98%, 99% identity to the corresponding variable domains, respectively.

29. The bispecific antibody according to any one of claims 12-26, consisting of a first heavy chain comprising the first antigen-binding domain and a second heavy chain comprising the second antigen-binding domain, and a first light chain; wherein the first heavy chain is selected from any one of the amino acid sequences shown in SEQ ID NOs: 69-74 or variants thereof, the second heavy chain has the sequence shown in SEQ ID NO: 68 or variants thereof, and the first light chain has the sequence shown in SEQ ID NO: 62 or variants thereof;
wherein the variants have 3, 2 or 1 amino acid differences from, or have at least 80%, 85%, 90%, 91%, 92%, 93%, 94%, 95%, 96%, 97%, 98%, 99% identity to the corresponding sequences, respectively.

30. The bispecific antibody according to any one of claims 12-29, wherein the bispecific antibody can simultaneously bind to EGFR and c-MET, and bind to EGFR and c-MET co-expressing cell lines with an EC₅₀ value lower than 10 nM.

31. The bispecific antibody according to any one of claims 12-29, wherein the bispecific antibody binds to EGFR high-expressing cell lines with an EC₅₀ value higher than 50 nM.

32. The bispecific antibody according to any one of claims 12-29, wherein the bispecific antibody is preferentially internalized by EGFR and c-MET co-expressing cell lines when EGFR and c-MET co-expressing cell lines and EGFR high-expressing cell lines are present simultaneously.

33. A nucleic acid molecule, encoding the antibody or antigen-binding fragment thereof according to any one of claims 1-10 or the multispecific antibody according to claim 11 or the bispecific antibody according to any one of claims 12-32.

34. An expression vector, comprising the nucleic acid molecule according to claim 33.

35. A host cell, comprising the nucleic acid molecule according to claim 33 or the expression vector according to claim 34, or expressing the antibody or antigen-binding fragment thereof according to any one of claims 1-10 or the multispecific antibody according to claim 11 or the bispecific antibody according to any one of claims 12-32.

36. An antibody-drug conjugate (ADC), consisting of an antibody, a linker and a payload, wherein the antibody is the antibody or antigen-binding fragment thereof according to any one of claims 1-10 or the multispecific antibody according to claim 11 or the bispecific antibody according to any one of claims 12-32.

37. A pharmaceutical composition, comprising the antibody or antigen-binding fragment thereof according to any one of claims 1-10 or the multispecific antibody according to claim 11 or the bispecific antibody according to any one of claims 12-32 or the antibody-drug conjugate according to claim 36, and at least one pharmaceutically acceptable carrier and/or excipient.

38. A method for inhibiting tumor cell growth and/or killing said tumor cells, comprising contacting said tumor cells with an effective amount of the antibody or antigen-binding fragment thereof according to any one of claims 1-10 or the multispecific antibody according to claim 11 or the bispecific antibody according to any one of claims 12-32 or the antibody-drug conjugate according to claim 36 or the pharmaceutical composition according to claim 37.

39. The antibody or antigen-binding fragment thereof according to any one of claims 1-10 or the multispecific antibody according to claim 11 or the bispecific antibody according to any one of claims 12-32 or the antibody-drug conjugate according to claim 36 or the pharmaceutical composition according to claim 37 for use in the manufacture of a medicament for the treatment of a tumor.

40. The use according to claim 39, wherein the tumor is selected from pancreatic cancer, melanoma, glioblastoma, head and neck cancer, prostate cancer, osteosarcoma, colorectal cancer, gastric cancer, malignant mesothelioma, multiple myeloma, ovarian cancer, non-small cell lung cancer, synovial sarcoma, thyroid cancer, breast cancer, squamous cell carcinoma, esophageal cancer, clear cell renal cell carcinoma, chromophobe renal cell carcinoma, renal oncocytoma, renal transitional cell carcinoma, urothelial carcinoma, adenocarcinoma or small cell carcinoma.
